# EUROPEAN PATENT SPECIFICATION

(11) **EP 4 149 603 B1**
(45) Date of publication and mention of the grant of the patent: **04.03.2026**
(21) Application number: 21730724.8
(22) Date of filing: 13.05.2021
(51) Int. Cl.: A61M 25/06, A61M 25/00

(54) **KINK-RESISTANT EXPANDABLE SHEATH**
KNICKBESTÄNDIGE DEHNBARE HÜLLE
GAINE EXPANSIBLE RÉSISTANTE AU PLIAGE

(30) Priority: 13.05.2020 US 202063024386 P
(43) Date of publication of application: 22.03.2023
(73) Proprietor: Edwards Lifesciences Corporation, Irvine, CA 92614 (US)
(72) Inventor: GUROVICH, Nikolay, 30889 Caesarea (IL); SHITRIT, Roy, 30889 Caesarea (IL); AVINATHAN, Itay, 30889 Caesarea (IL)
(74) Representative: Eisenführ Speiser
(86) International application number: PCT/US2021/032271
(87) International publication number: WO 2021/231743

(56) References cited:
- EP-A1- 2 995 268
- WO-A1-2019/212814

## Description

### CROSS-REFERENCE TO RELATED APPLICATIONS

This application claims the benefit of U.S. Provisional Application No.63/024,386, filed May 13, 2020.

### FIELD

The present application relates to expandable introducer sheaths for prosthetic devices such as transcatheter heart valves and methods of making the same.

### BACKGROUND

Endovascular delivery catheter assemblies are used to implant prosthetic devices, such as a prosthetic valve, at locations inside the body that are not readily accessible by surgery or where access without invasive surgery is desirable. For example, aortic, mitral, tricuspid, and/or pulmonary prosthetic valves can be delivered to a treatment site using minimally invasive surgical techniques.

An introducer sheath can be used to safely introduce a delivery apparatus into a patient's vasculature (e.g., the femoral artery). An introducer sheath generally has an elongated sleeve that is inserted into the vasculature and a housing that contains one or more sealing valves that allow a delivery apparatus to be placed in fluid communication with the vasculature with minimal blood loss. Such introducer sheaths may be radially expandable. However, such sheaths tend to have complex mechanisms, such as ratcheting mechanisms that maintain the sheath in an expanded configuration once a device with a larger diameter than the sheath's original diameter is introduced. Existing expandable sheaths can also be prone to the kinks as a consequence of the application of longitudinal force attendant to passing a prosthetic device through the sheath, especially during passing through a curved vasculature of various radii. As a result, an increased force is required to insert the prosthetic device through the narrowed sheath.

EP 2 995 268 A1 discloses an expandable introducer sheath for use in inserting a medical device into a body vessel of a patient including a body which extends from a proximal end to a distal end along an axis. The body includes an inner layer, an outer layer, and an expandable reinforcement member which is disposed between said inner and outer layers. The expandable reinforcement member is configured to radially expand as the medical device is axially advanced or retracted through said introducer sheath. Once the medical device has exited the introducer sheath, the expandable reinforcement member facilitates a return of the introducer sheath to its original or unexpanded condition.

WO 2019/212814 A1 is directed to reinforced expandable introducer sheaths and methods of using the same. The sheaths minimize trauma to the patient's vasculature by including a reinforcing member to improve the push force of the sheath while reducing the chances of kinking during delivery of a prosthetic device. The sheath can include a plurality of expandable rings aligned along the longitudinal axis of the sheath and coupled together to form an elongated tubular structure. The sheath can also include a plurality of radial members circumferentially arranged to form a tubular support structure. The radial members are slidingly interconnected around the circumference of the tubular structure to facilitate radial expansion of the sheath.

Accordingly, there remains a need in the art for an improved introducer sheath for endovascular systems used for implanting valves and other prosthetic devices. Still, further, there is a need for introducer sheaths that exhibit a reduced insertion force as compared to conventional introducers. Also, there is still a need for methods of making the same. In the following, 1 inch can be converted into 25,4mm.

### SUMMARY

The aspects of the present disclosure relate to the kink-resistant expandable sheaths for deploying a medical device. The expandable sheaths for deploying a medical device comprises at least one plurality of struts arranged to form an expandable cylindrical frame having a proximal and distal end, an inner surface and an outer surface, and a central axis extending along a length of the frame; wherein the expandable cylindrical frame has a first length and an opposite second length, and wherein, in a first position, at least a portion of the first length and at least a portion of the second length are substantially the same; wherein the expandable cylindrical frame is movable from the first position to a second position by curving away from the central axis, wherein at the second position at least a portion of the first length shortens and at least a portion of the second length elongates; wherein the inner surface of the expandable cylindrical frame defines a lumen configured to receive a medical device; and wherein the expandable cylindrical frame is configured to expand from a first diameter d₁ in an unexpended position to a second diameter d₂ an expanded position upon passage of a medical device.

In yet other aspects, the portion of the first length and the portion of the second length in the second position are located along a curved portion of the frame. In yet further aspects, the first length is provided on a first portion of an outer diameter of the expandable cylindrical frame, and the second length is provided on a second portion of the outer diameter of the expandable cylindrical frame, wherein the first portion is circumferentially opposite to the second portion of the outer diameter.

In yet still further aspects, the expandable cylindrical sheath of any one of the disclosed aspects is kink resistant.

In some aspects, the plurality of struts can be arranged to form a zig-zag configuration comprising a plurality of substantially straight portions circumferentially followed by a plurality of substantially bent portions and wherein the zig-zag configuration is helically wound along a central axis to form the frame. In some exemplary aspects, each substantially straight portion of the plurality of substantially straight portions being of equal lengths. While in other exemplary aspects, the plurality of substantially straight portions can comprise one or more portions having unequal lengths at the distal and/or proximal ends of the frame.

Still further described herein are aspects where the plurality of struts are arranged in a zig-zag configuration to form a plurality of parallel circumferential rows, wherein each row of the plurality of parallel circumferential rows comprises a plurality of substantially straight portions circumferentially followed by a plurality of substantially bent portions; and wherein the plurality of parallel circumferential rows are axially coupled to each other by a singular vertical strut member disposed along a length of the frame of the medical device, the frame does not expand along the singular vertical strut member.

Also described herein are aspects where the plurality of struts are arranged in a configuration wherein each strut of the plurality of struts is able to individually elongate in a circumferential direction along the length of the frame upon introduction of the medical device.

Still further disclosed are the aspects where the plurality of struts are arranged in a zig-zag configuration to form a plurality of parallel circumferential rows, wherein each row of the plurality of parallel circumferential rows comprises a plurality of substantially straight portions circumferentially followed by a plurality of substantially bent portions; wherein adjacent rows of the plurality of parallel circumferential rows are coupled together by a singular bridging member extending from a substantially bent portion in a first row to a corresponding substantially bent portion in an adjacent second row, and wherein the bridging member coupling any two adjacent rows is circumferentially offset from a bridging member coupling any following two adjacent rows.

Also disclosed are the aspects where the cylindrical frame is formed by a first plurality of struts arranged along a first edge of a singular vertical member and a second plurality of struts arranged along an opposite second edge of the singular vertical member, wherein each strut of the first plurality of struts is spaced from each other such that a first gap is formed between two adjacent struts in the first plurality of struts; wherein each strut of the second plurality of struts is spaced from each other such that a second gap is formed between two adjacent struts in the second plurality of struts; wherein each strut of the first plurality of struts is axially offset along the singular vertical member from each strut of the second plurality of struts; and wherein in the unexpanded position, each strut of the first plurality of struts is disposed within the second gap, and each strut of the second plurality of struts is disposed within the first gap.

Still further, disclosed herein are the aspects where the sheath further comprises one or more polymeric layers. In such exemplary aspects, the polymeric layer comprises one or more of polyethylene, polypropylene, polyether block amide (PEBAX^{®}), silicon, or any combination thereof.

In still further aspects, at least a portion of the frame is embedded within the one or more polymeric layers. In certain exemplary aspects, the one or more polymeric layers comprise a plurality of longitudinally extending folds wherein the sheath is at the first diameter d₁. While in other exemplary aspects, the longitudinally extending folds create a plurality of circumferentially spaced ridges and a plurality of circumferentially spaced valleys. In still further aspects, as the medical device passes through the sheath, the ridges and valleys can level out to allow the sheath to radially expand.

Methods of making expandable sheaths are also disclosed herein. One aspect of a method comprises arranging at least one plurality of struts to form an expandable cylindrical frame having a proximal and distal end, an inner surface and an outer surface, and a central axis extending along a length of the frame, wherein the expandable cylindrical frame has a first length and an opposite second length, and wherein in a first position at least a portion of the first length and at least a portion of the second length are substantially the same, wherein the expandable cylindrical frame is movable from the first position to a second position by curving away from the central axis, wherein at the second position at least a portion of the first length shortens and at least a portion of the second length elongates; wherein the inner surface of the expandable cylindrical frame defines a lumen configured to receive a medical device; and wherein the expandable cylindrical frame is configured to expand from a first diameter d₁ in an unexpended position to a second diameter d₂ an expanded position upon passage of a medical device.

Still further disclosed are the aspects describing the methods further comprising a step of forming a pre-shaped flare at the distal end of the frame such that the pre-shaped flare is configured to stay compressed at the rest position and to assume a flared configuration upon passing the medical device. In such exemplary aspects, the pre-shaped flare is kept compressed by attaching a filament one or more of the plurality of struts at the distal end of the frame and extending it circumferentially around the plurality of struts at the distal end. In certain aspects, when the distal end of the frame is in the expanded position, the filament is configured to stay attached to one or more of the plurality of struts at the distal end of the frame without extending circumferentially around the plurality of struts at the distal end thereby allowing the distal end of the frame to flare radially outward.

It is understood that the methods disclosed herein include aspects where the medical device is a cardiovascular device. In such aspects, the cardiovascular device can include at least one of prosthetic heart valve and a stent. In still further aspects, disclosed herein methods comprise applying an outer layer on the sheath.

Additional aspects of the disclosure will be set forth, in part, in the detailed description, figures, and claims which follow, and in part will be derived from the detailed description or can be learned by practice of the invention. It is to be understood that both the foregoing general description and the following detailed description are exemplary and explanatory only and are not restrictive of the invention as disclosed.

### BRIEF DESCRIPTION OF THE DRAWINGS

**FIG. 1** illustrates a delivery system for a cardiovascular prosthetic device, according to one exemplary aspect.
**FIG. 2** illustrates an expandable sheath that can be used in combination with the delivery system of **FIG.** 1, according to one exemplary aspect.
**FIG.** 3 illustrates an expandable sheath in one exemplary aspect.
**FIG. 4** is an exemplary frame in one exemplary aspect.
**FIG. 5** is a magnified view of a distal portion of the frame of **FIG. 4****,** according to one exemplary aspect.
**FIG. 6** is a magnified view of a mid-portion of the frame of **FIG. 4****,** according to one exemplary aspect.
**FIG. 7** is a flattened view of a distal portion of the frame of **FIG. 4****,** according to one exemplary aspect.
**FIG. 8** is an exemplary frame of a distal portion of the frame in one exemplary aspect.
**FIG. 9** is a magnified view of a mid-portion of the frame of **FIG. 8****,** according to one exemplary aspect.
**FIG. 10** is a flattened view of a distal portion of the frame of **FIG. 8****,** according to one exemplary aspect.
**FIG. 11** is a flattened view of a mid-portion portion of the frame of **FIG. 9****,** according to one exemplary aspect.
**FIG. 12** is an exemplary frame in one exemplary aspect.
**FIG. 13** is a magnified view of a distal portion of the frame of **FIG. 12****.**
**FIG. 14** is a magnified view of a mid-portion of the frame of **FIG. 12****.**
**FIG. 15** is a flattened view of a distal portion of the frame of **FIG. 13****,** according to one exemplary aspect.
**FIG. 16** is an exemplary frame in one exemplary aspect.
**FIG. 17** is a magnified view of a distal portion of the frame of **FIG. 16****.**
**FIG. 18** is a magnified view of a mid-portion of the frame of **FIG. 16****.**
**FIG. 19** is a flattened view of a distal portion of the frame of **FIG. 17****,** according to one exemplary aspect.
**FIG. 20A** is an exemplary frame in one exemplary aspect.
**FIG. 20B** is a back perspective view of the frame of **FIG. 20****.**
**FIG. 21** is a flattened view of the frame of **FIG. 20****.**
**FIGS. 22A-22F** depict simulated insertion and removal of a medical device through an exemplary frame of **FIG. 20A** in one exemplary aspect.
**FIGS. 23A-23D** depict simulated insertion and removal of a medical device through an exemplary frame of **FIG. 20A** in one exemplary aspect.
**FIGS. 24A-24G** depict simulated insertion and removal of a medical device through an exemplary frame of **FIG. 20A** in one exemplary aspect.
**FIGS. 25A-25F** depict simulated insertion and removal of a medical device through an exemplary frame of **FIG. 20A** in one exemplary aspect.
**FIGS. 26A-26F** depict simulated insertion and removal of a medical device through an exemplary frame of **FIG. 20A** in one exemplary aspect.
**FIG. 27** are photographs of an exemplary sheath demonstrating kink resistance upon passing and removal of a medical device in conduits having a curve of the various radii.
**FIG. 28** depicts an exemplary distal portion of a frame in one exemplary aspect with a filament circumferentially attached to the frame.
**FIG. 29** depicts an exemplary distal portion of a frame in one exemplary aspect after the passage of a medical device.
**FIGS. 30A-30B** show exemplary ridges and valleys formed in an exemplary polymeric material.

### DETAILED DESCRIPTION

The present disclosure can be understood more readily by reference to the following detailed description, examples, drawings, and claims, and their previous and following description. However, before the present articles, systems, and/or methods are disclosed and described, it is to be understood that this disclosure is not limited to the specific or exemplary aspects of articles, systems, and/or methods disclosed unless otherwise specified, as such can, of course, vary. It is also to be understood that the terminology used herein is for the purpose of describing particular aspects only and is not intended to be limiting.

The following description of the disclosure is provided as an enabling teaching of the disclosure in its best, currently known aspect. To this end, those skilled in the relevant art will recognize and appreciate that many changes can be made to the various aspects of the disclosure described herein while still obtaining the beneficial results of the present disclosure. It will also be apparent that some of the desired benefits of the present disclosure can be obtained by selecting some of the features of the present disclosure without utilizing other features. Accordingly, those of ordinary skill in the pertinent art will recognize that many modifications and adaptations to the present disclosure are possible and may even be desirable in certain circumstances and are a part of the present disclosure. Thus, the following description is again provided as illustrative of the principles of the present disclosure and not in limitation thereof.

### DEFINITIONS

As used in this application and in the claims, the singular forms "a," "an," and "the" include the plural forms unless the context clearly dictates otherwise. Thus, for example, reference to a "frame" includes aspects having two or more such frames unless the context clearly indicates otherwise.

It is also to be understood that the terminology used herein is for the purpose of describing particular aspects only and is not intended to be limiting. As used in the specification and in the claims, the term "comprising" can include the aspects "consisting of" and "consisting essentially of."

Unless defined otherwise, all technical and scientific terms used herein have the same meaning as commonly understood by one of ordinary skill in the art to which this disclosure belongs. In this specification and in the claims, which follow, reference will be made to a number of terms that shall be defined herein.
For the terms "for example" and "such as," and grammatical equivalences thereof, the phrase "and without limitation" is understood to follow unless explicitly stated otherwise.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements. Furthermore, when numerical ranges of varying scope are set forth herein, it is contemplated that any combination of these values inclusive of the recited values may be used. Further, ranges can be expressed herein as from "about" one particular value and/or to "about" another particular value. When such a range is expressed, another aspect includes from the one particular value and/or to the other particular value.

Similarly, when values are expressed as approximations, by use of the antecedent "about," it will be understood that the particular value forms another aspect. It will be further understood that the endpoints of each of the ranges are significant both in relation to the other endpoint and independently of the other endpoint. Unless stated otherwise, the term "about" means within 5% (e.g., within 2% or 1%) of the particular value modified by the term "about."

Throughout this disclosure, various aspects of the disclosure can be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6, etc., as well as individual numbers within that range, for example, 1, 2, 2.7, 3, 4, 5, 5.3, 6 and any whole and partial increments therebetween. This applies regardless of the breadth of the range.

It will be understood that when an element is referred to as being "connected" or "coupled" to another element, it can be directly connected or coupled to the other element, or intervening elements may be present. In contrast, when an element is referred to as being "directly connected" or "directly coupled" to another element, there are no intervening elements present. Other words used to describe the relationship between elements or layers should be interpreted in a like fashion (e.g., "between" versus "directly between," "adjacent" versus "directly adjacent," "on" versus "directly on"). As used herein, the term "and/or" includes any and all combinations of one or more of the associated listed items.

It will be understood that, although the terms "first," "second," etc., may be used herein to describe various elements, components, regions, layers and/or sections. These elements, components, regions, layers, and/or sections should not be limited by these terms. These terms are only used to distinguish one element, component, region, layer, or section from another element, component, region, layer, or a section. Thus, a first element, component, region, layer, or section discussed below could be termed a second element, component, region, layer, or section without departing from the teachings of exemplary aspects.

Spatially relative terms, such as "beneath," "below," "lower," "above," "upper," and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if the device in the figures is turned over, elements described as "below" or "beneath" other elements or features would then be oriented "above" the other elements or features. Thus, the term "below" can encompass both an orientation of above and below. The device may be otherwise oriented (rotated 90 degrees or at other orientations), and the spatially relative descriptors used herein are interpreted accordingly.

As used herein, the term "substantially" means that the subsequently described event or circumstance completely occurs or that the subsequently described event or circumstance generally, typically, or approximately occurs.

Still further, the term "substantially" can in some aspects refer to at least about 80 %, at least about 85 %, at least about 90 %, at least about 91 %, at least about 92 %, at least about 93 %, at least about 94 %, at least about 95 %, at least about 96 %, at least about 97 %, at least about 98 %, at least about 99 %, or about 100 % of the stated property, component, composition, or other condition for which substantially is used to characterize or otherwise quantify an amount.

In other aspects, as used herein, the term "substantially free," when used in the context of a surface substantially free of defects, for example, is intended to refer to a surface that has less than about 5 % of defects, less than about 4.5 % of defects, less than about 4 % of defects, less than about 3.5 % of defects, less than about 3 % of defects, less than about 2.5 % of defects, less than about 2 % of defects, less than about 1.5 % of defects, less than about 1 % of defects, less than about 0.5 % of defects, less than about 0.1 % of defects, less than about 0.05 % of defects, or less than about 0.01 % of defects of the total surface.

As used herein, the term "substantially," in, for example, the context "substantially no change" refers to a phenomenon or an event that exhibits less than about 1 % change, e.g., less than about 0.5 %, less than about 0.1 %, less than about 0.05 %, or less than about 0.01 % change.

As used herein, the term "substantially," in, for example, the context "substantially identical" or "substantially similar" refers to a method or a system, or a component that is at least about 80%, at least about 85%, at least about 90%, at least about 91%, at least about 92%, at least about 93%, at least about 94%, at least about 95%, at least about 96%, at least about 97%, at least about 98%, at least about 99%, or about 100% by similar to the method, system, or the component it is compared to.

As used herein, the term "atraumatic" is commonly known in the art and refers to a device or a procedure that minimized tissue injury.

As used herein, the term or phrase "effective," "effective amount," or "conditions effective to" refers to such amount or condition that is capable of performing the function or property for which an effective amount or condition is expressed. As will be pointed out below, the exact amount or particular condition required will vary from one aspect to another, depending on recognized variables such as the materials employed and the processing conditions observed. Thus, it is not always possible to specify an exact "effective amount" or "condition effective to." However, it should be understood that an appropriate effective amount will be readily determined by one of ordinary skill in the art using only routine experimentation.

Although the operations of exemplary aspects of the disclosed method may be described in a particular, sequential order for convenient presentation, it should be understood that disclosed aspects can encompass an order of operations other than the particular, sequential order disclosed. For example, operations described sequentially may, in some cases, be rearranged or performed concurrently. Further, descriptions and disclosures provided in association with one particular aspect are not limited to that aspect and may be applied to any aspect disclosed.

Moreover, for the sake of simplicity, the attached figures may not show the various ways (readily discernable, based on this disclosure, by one of ordinary skill in the art) in which the disclosed system, method, and apparatus can be used in combination with other systems, methods, and apparatuses. Additionally, the description sometimes uses terms such as "produce" and "provide" to describe the disclosed method. These terms are high-level abstractions of the actual operations that can be performed. The actual operations that correspond to these terms can vary depending on the particular implementation and are, based on this disclosure, readily discernible by one of ordinary skill in the art.

### SHEATH

The expandable introducer sheaths described herein can be used to deliver a prosthetic device through a patient's vasculature to a procedure site within the body. The sheath can be constructed to be highly expandable and collapsible in the radial direction while limiting axial elongation of the sheath and, thereby, undesirable narrowing of the lumen. In still further aspects and as described herein, the sheath is substantially kink-free.

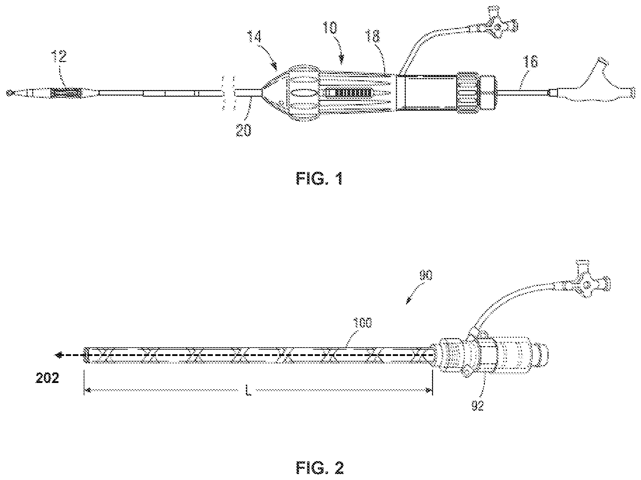

**FIG. 1** illustrates a representative delivery apparatus **10** for delivering a medical device, such as a prosthetic heart valve or other prosthetic implant, to a patient. The delivery apparatus **10** is exemplary only and can be used in combination with any of the expandable sheaths as described herein. Likewise, the sheaths disclosed herein can be used in combination with any of the various known delivery apparatuses. The delivery apparatus **10** illustrated herein can generally include a steerable guide catheter **14** and a balloon catheter **16** extending through the guide catheter **14.** A prosthetic device, such as a prosthetic heart valve **12,** can be positioned on the distal end of the balloon catheter **16.** The guide catheter **14** and the balloon catheter **16** can be adapted to slide longitudinally relative to each other to facilitate delivery and positioning of a prosthetic heart valve **12** at an implantation site in a patient's body. The guide catheter **14** includes a handle portion **18** and an elongated guide tube or shaft **20** extending from the handle portion **18.**

The prosthetic heart valve **12** can be delivered into a patient's body in a radially compressed configuration and radially expanded to a radially expanded configuration at the desired deployment site. In some exemplary aspects, the prosthetic heart valve **12** is a plastically expandable prosthetic valve that is delivered into the patient's body in a radially compressed configuration on a balloon of the balloon catheter **16** (as shown in **FIG. 1****)** and then radially expanded to a radially expanded configuration at the deployment site by inflating the balloon (or by actuating another type of expansion device of the delivery apparatus). Further details regarding a plastically expandable heart valve that can be implanted using the devices disclosed herein are disclosed in U.S. Publication No. 2012/0123529. In other aspects, the prosthetic heart valve **12** can be a self-expandable heart valve that is restrained in a radially compressed configuration by a sheath or other component of the delivery apparatus and self-expands to a radially expanded configuration when released by the sheath or other component of the delivery apparatus. Further details regarding a self-expandable heart valve that can be implanted using the devices disclosed herein are disclosed in U.S. Publication No. 2012/0239142. In still other aspects, the prosthetic heart valve **12** can be a mechanically expandable heart valve that comprises a plurality of struts connected by hinges or pivot joints and is expandable from a radially compressed configuration to a radially expanded configuration by actuating an expansion mechanism that applies an expansion force to the prosthetic valve. Further details regarding a mechanically expandable heart valve that can be implanted using the devices disclosed herein are disclosed in U.S. Publication No. 2018/0153689. In still other aspects, a prosthetic valve can incorporate two or more of the above-described technologies. For example, a self-expandable heart valve can be used in combination with an expansion device to assist expansion of the prosthetic heart valve.

**FIG. 2** illustrates an assembly **90** (which can be referred to as an introducer device or assembly) that can be used to introduce the delivery apparatus **10** and the prosthetic device **12** into a patient's body, according to one aspect. The introducer device **90** can comprise a housing **92** at a proximal end of the device and an expandable sheath **100** extending distally from the housing **92.** The housing **92** can function as a handle for the device. Generally, during use, a distal end of the sheath **100** is passed through the skin of the patient and is inserted into a vessel, such as the femoral artery. The delivery apparatus **10** with its implant **12** can then be inserted through the housing **92** and the sheath **100,** and advanced through the patient's vasculature to the treatment site, where the implant is to be delivered and implanted within the patient. In certain aspects, the introducer housing **92** can include a hemostasis valve that forms a seal around the outer surface of the guide catheter **14** once inserted through the housing to prevent leakage of pressurized blood.

In alternative aspects, the introducer device **90** need not include a housing **92.** For example, the disclosed sheath **100** can be an integral part of a component of the delivery apparatus **10,** such as the guide catheter. For example, the sheath can extend from the handle **18** of the guide catheter. Additional examples of introducer devices and expandable sheaths can be found in U.S. Patent Application No. 16/378,417.

In still alternative aspects, the disclosed herein sheath **100** can be used with the introducer device **90** to deliver a stent or other implantable device. In still further aspects, the introducer device **90** having the disclosed sheath **100** can also be used to deliver non-self-expanding prostheses such as a balloon-expandable stent. Additional examples of introducer devices for the introduction of the stents are found in U.S. Patent No. 6,344,044.

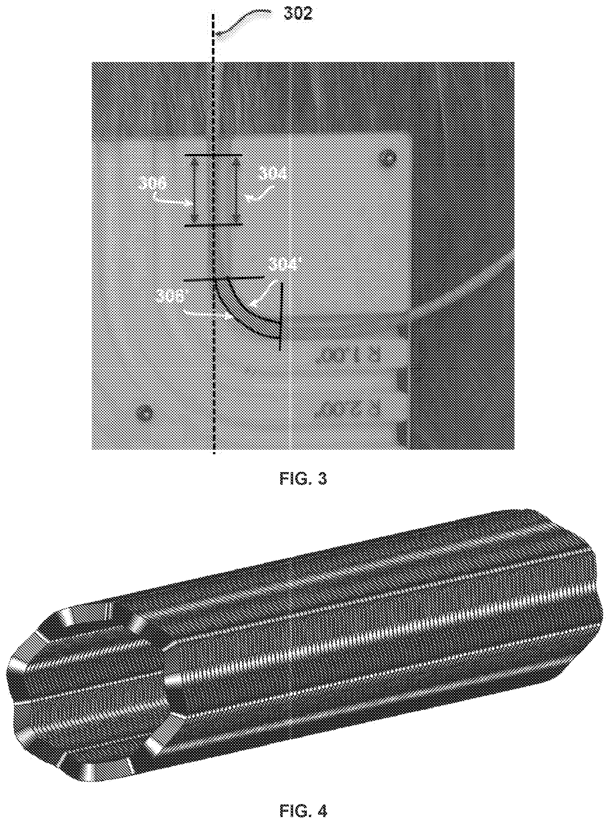

In still further aspects, disclosed herein is an expandable sheath for deploying a medical device comprising at least one plurality of struts arranged to form an expandable cylindrical frame having a proximal and distal end, an inner surface and an outer surface, and a central axis extending along a length of the frame; wherein the expandable cylindrical frame has a first length and an opposite second length, and wherein in a first position at least a portion of the first length and at least a portion of the second length are substantially the same; wherein the expandable cylindrical frame is movable from the first position to a second position by curving away from the central axis, wherein at the second position at least a portion of the first length shortens and at least a portion of the second length elongates; wherein the inner surface of the expandable cylindrical frame defines a lumen configured to receive a medical device; and wherein the expandable cylindrical frame is configured to expand from a first diameter d₁ in an unexpanded position to a second diameter d₂ an expanded position upon passage of a medical device. **FIG. 3** shows an exemplary photograph of the disclosed sheath. The expandable sheath **300** shown in **FIG. 3** comprises a frame and a polymeric material whose configurations are discussed in detail below. The expandable cylindrical frame has a central axis **302** and a first length and an opposite second length. As can be seen in **FIG. 3****,** in a first position, at least a portion of the first length **304** and at least a portion of the second length **306** are substantially the same. It can be further seen that when the frame (and as a result, the sheath) moves from the first position to the second position by curving away from the central axis the at least a portion of the first length **304'** shortens, while at least a portion of the second length **306'** elongates. In still further aspects, the first length is provided on a first portion of an outer diameter of the expandable cylindrical frame, and the second length is provided on a second portion of the outer diameter of the expandable cylindrical frame, wherein the first portion is circumferentially opposite to the second portion around the outer diameter. Without wishing to be bound by any theory, it is hypothesized that in certain and unlimiting aspects, this configuration allows the frame (and as a result, the sheath) to be substantially kink-resistant when passing through the curved and folded pathways of the patient's vasculature.

In certain aspects, the first diameter d₁ of the frame in the unexpanded position can be from about 3 mm to about 5 mm, including exemplary values of about 3.2 mm, about 3.5 mm, about 3.7 mm, about 4 mm, about 4.2 mm, and about 4.8 mm. It is further understood that the first diameter d₁ can have any value between any two foregoing values, for example, and without limitation, the first diameter d₁ can be between 3 mm and 4 mm, or between 3.5 mm and 4.5 mm.

In yet other aspects, the second diameter d₂ of the frame in the expanded position can be from about 5 mm to about 10 mm, including exemplary values of about 5.2 mm, about 5.5 mm, about 5.7 mm, about 6 mm, about 6.2 mm, about 6.5 mm, about 6.7 mm, about 7 mm, 7.2 mm, about 7.5 mm, about 7.7 mm, about 8 mm, about 8.2 mm, about 8.5 mm, about 8.7 mm, about 9 mm, 9.2 mm, about 9.5 mm, and about 9.7 mm. It is further understood that the second diameter d₂ can have any value between any two foregoing values, for example, and without limitation, the second diameter d₂ can be between 5 mm and 6 mm, or between 5 mm and 8 mm.

In still further aspects, the frame disclosed herein is configured to contract to a diameter that is substantially the same as the first diameter d₁ upon removal and/or passage of the medical device. In such aspects, for example, and without limitation, when the first diameter d₁ expands upon passage of the medical device from about 4 mm to the second diameter d₂ of about 6 mm, the frame is configured to contract to a diameter substantially identical to about 4 mm. However, in some aspects, when expansion of the frame is larger due to the passage of the larger medical device, the return contraction to the first diameter can be not favorable, as it can result in hemorrhage and undesired risk to the patient. In such exemplary aspects, the frame can be configured to contract to a diameter that is from about 10% to about 90%, including exemplary values of about 15%, about 20%, about 25%, about 30%, about 35%, about 40%, about 45%, about 50%, about 55%, about 60%, about 65%, about 70%, about 75%, about 80%, and about 85% larger than the first diameter d₁ upon removal of the medical device. For example, and without limitation, when the first diameter d₁ expands upon passage of the medical device from about 4 mm to the second diameter d₂ of about 8 mm, the frame can be configured to contract to a diameter of about 6 mm, to prevent hemorrhage.

In still further aspects and as disclosed herein, the frame can have the first diameter d₁ that is substantially uniform along the length of the frame. However, in some exemplary aspects, the first diameter d₁ can vary along the length of the frame. In such exemplary aspects, the first diameter d₁ at the proximal end, where the frame (sheath) connects to the housing **92** (as shown in **FIG.2****),** can be larger than the first diameter d₁ at the distal end. In yet further aspects, the diameter d₁ can gradually decrease from the proximal end of the frame to the middle portion of the frame and then decrease again towards the distal end of the frame.

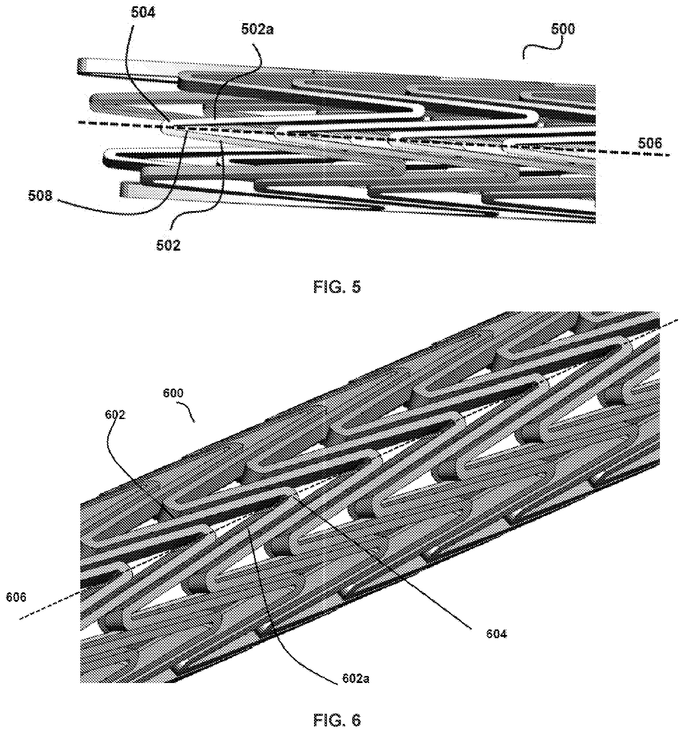

**FIG. 4-7** show an exemplary frame, as disclosed in some aspects described herein. **FIG.** 4 shows an exemplary 3D schematic of the frame in the expanded configuration. **FIGS. 5** and **6** show a more detailed configuration of the exemplary frame **500** at a distal end and the exemplary frame **600** in the middle of the sheath, respectively. In these exemplary aspects, the plurality of struts are arranged to form a zig-zag configuration comprising a plurality of substantially straight portions **502** and **502a** or **602** and **602a** circumferentially followed by a plurality of substantially bent portions **504** or **604** and wherein the zig-zag configuration is wound along the central axis to form the frame. In certain aspects, the zig-zag configuration is helically wound along the central axis to form the frame. It is understood that in certain aspects, the plurality of substantially straight portions **(502, 502a, 602, 602a)** can have an equal predetermined length. While in other aspects, the plurality of substantially straight portions **(502, 502a, 602, 602a)** can comprise one or more portions that can have unequal lengths, for example, at the distal and/or proximal ends of the frame to accommodate optional variations in diameter d₁ as described above. It is further understood that the length of each substantially straight portion of the plurality of substantially straight portions **(502, 502a, 602, 602a)** can be easily determined depending on the desired application and to what extent the frame is expected to expand. It can also be seen in **FIG. 5** and **FIG. 6** that the plurality of substantially bent portions **(504, 604)** can define a bisecting line **506, 606** extending through an apex of the plurality of substantially bent portions **(504, 604)** and extending longitudinally along a length of the sheath. In such aspects, each substantially straight portion of the plurality of substantially straight portions **(502, 502a, 602, 602a)** can form a first angle **508** with the bisecting line from 0° to about 90°, including exemplary angles of about 5°, about 10°, about 15 °, about 20°, about 25°, about 30°, about 35°, about 40°, about 45°, about 50°, about 55°, about 60°, about 65°, about 70°, about 75°, about 80°, and about 85°.

In yet further aspects, it is understood that when the frame is in the expanded position, the first angle increases during the expansion by greater than 0% to about 1,000% of its original value, including exemplary value of about 10%, about 20%, about 30%, about 40%, about 50%, about 60%, about 70%, about 80%, about 90%, about 100%, about 150%, about 200%, about 250%, about 300%, about 350%, about 400%, about 450%, about 500%, about 550%, about 600%, about 650%, about 700%, about 750%, about 800%, about 850%, about 900%, and about 950%. In yet further aspects, it is understood that the first angle can be increased by any value between any two foregoing values, for example, the first angle can be increased by greater than 0% to about 600% of its original value, or by 10% to about 400%, or by 100% to about 800%.

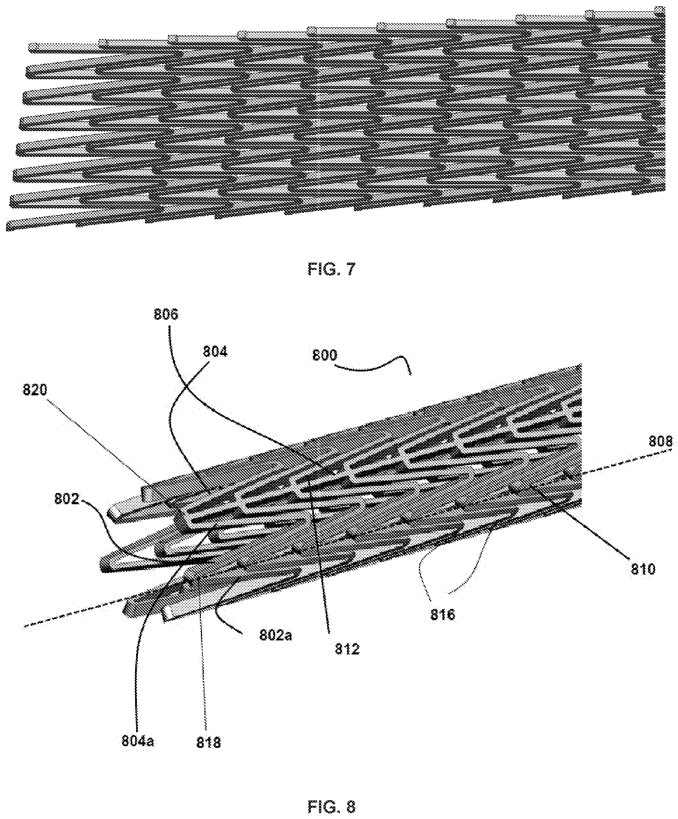

**FIG. 7** shows a planar schematic of the frame configuration described in this aspect.

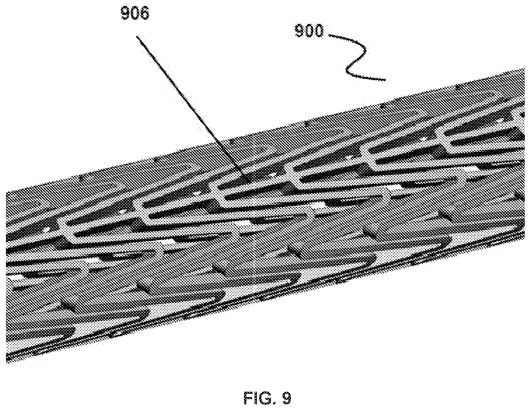

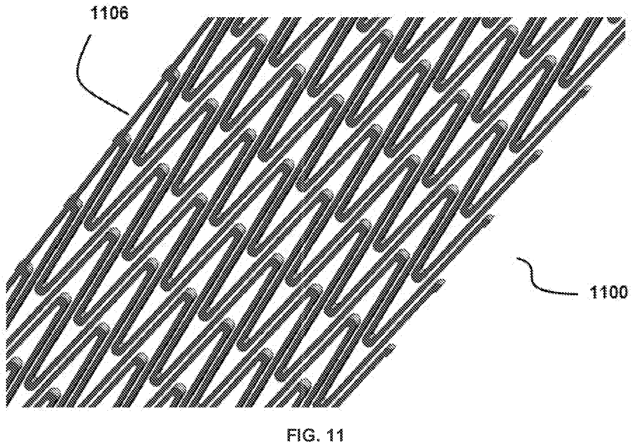

**FIGS. 8-11** show an additional frame configuration according to other aspects of the disclosure. For example, **FIGS. 8** and **9** show frame configurations **800** and **900** at the distal end and in the middle of the frame, respectively. In this exemplary aspect, the plurality of struts are arranged in a zig-zag configuration to form a plurality of parallel circumferential rows **816,** wherein each row of the plurality of parallel circumferential rows **816** comprises a plurality of substantially straight portions **802** and **802a** circumferentially followed by a plurality of substantially bent portions **818;** and wherein the plurality of parallel circumferential rows **816** are axially coupled to each other by a singular vertical strut member **806** disposed along a length of the frame **800, 900.** In some aspects, each substantially straight portion of the plurality of substantially straight portions **802, 802a** can have equal lengths in this configuration; while in other aspects, the plurality of substantially straight portions **802, 802a** can comprise one or more portions having unequal lengths, e.g., at the distal and/or proximal ends of the frame. Also, in some aspects, the plurality of substantially bent portions **818** can define a bisecting line **808** extending through an apex of the plurality of substantially bent portions **818** and extending longitudinally along a length of the sheath. In such aspects, each substantially straight portion of the plurality of substantially straight portions **802, 802a** can form a first angle **810** with the bisecting line **808** from 0° to about 90°, including exemplary angles of about 5°, about 10°, about 15°, about 20°, about 25°, about 30°, about 35°, about 40°, about 45°, about 50°, about 55°, about 60°, about 65°, about 70°, about 75°, about 80°, and about 85°. In yet further aspects, it is understood that when the frame is in the expanded position, the first angle increases during the expansion by greater than 0% to about 1,000% of its original value, including exemplary value of about 10%, about 20%, about 30%, about 40%, about 50%, about 60%, about 70%, about 80%, about 90%, about 100%, about 150%, about 200%, about 250%, about 300%, about 350%, about 400%, about 450%, about 500%, about 550%, about 600%, about 650%, about 700%, about 750%, about 800%, about 850%, about 900%, and about 950%. In yet further aspects, it is understood that the first angle can be increased by any value between any two foregoing values, for example, the first angle can be increased by greater than 0% to about 600% of its original value, or by 10% to about 400%, or by 100% to about 800%.

In further aspects and as shown in **FIG. 8****,** a singular vertical strut member **806** can extend longitudinally along a length of the sheath and connect apexes **820** of the substantially bending portions **818** of each row of the plurality of parallel circumferential rows **816.** In such exemplary aspects, the vertical strut member **806** can form a second angle **812** with each substantially straight portion of the corresponding adjacent substantially straight portions **804, 804a** of 0° to about 90°, including exemplary angles of about 5 °, about 10 °, about 15 °, about 20 °, about 25°, about 30°, about 35°, about 40°, about 45°, about 50°, about 55°, about 60°, about 65°, about 70°, about 75°, about 80°, and about 85°. In certain aspects, when the medical device is introduced into the frame as disclosed herein, the frame expands circumferentially in a direction opposite to the single vertical strut member **806** while not expanding along the singular vertical strut member **806.** In this exemplary aspect, the frame can have a "rod-like" behavior, able to substantially bend in any direction without kinking.

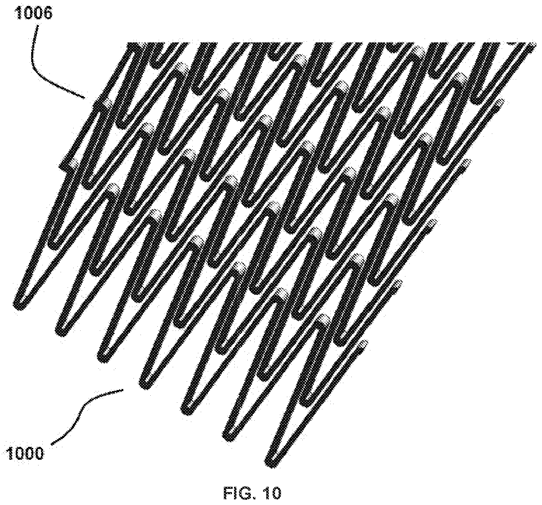

**FIGS. 10-11** show a flattened view of various sections of the frame configuration described in the exemplary aspects having the singular vertical strut member **1006** and **1106,** respectively.

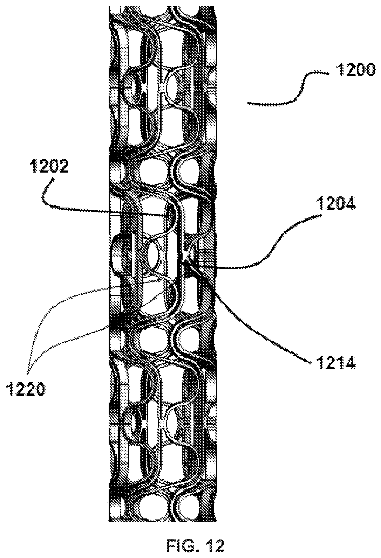

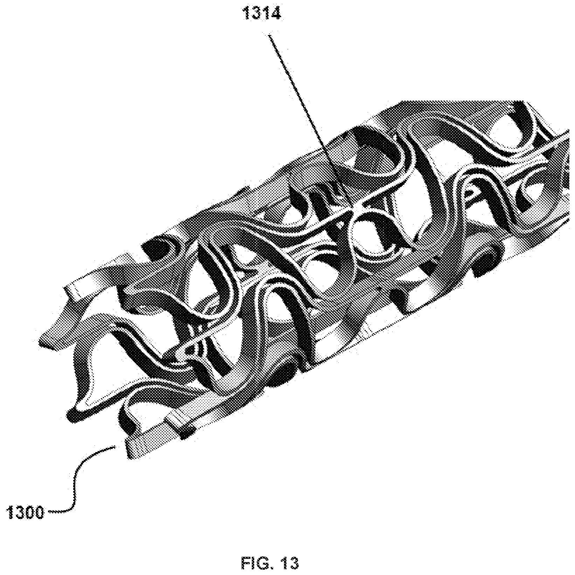

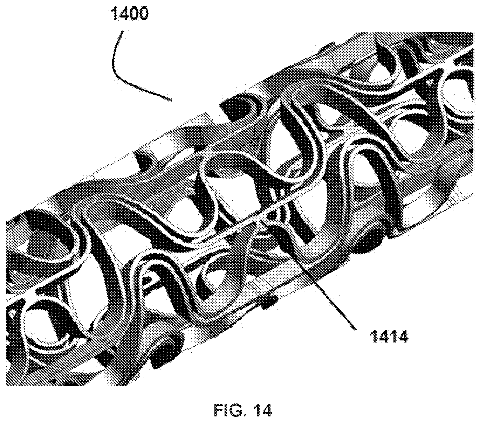

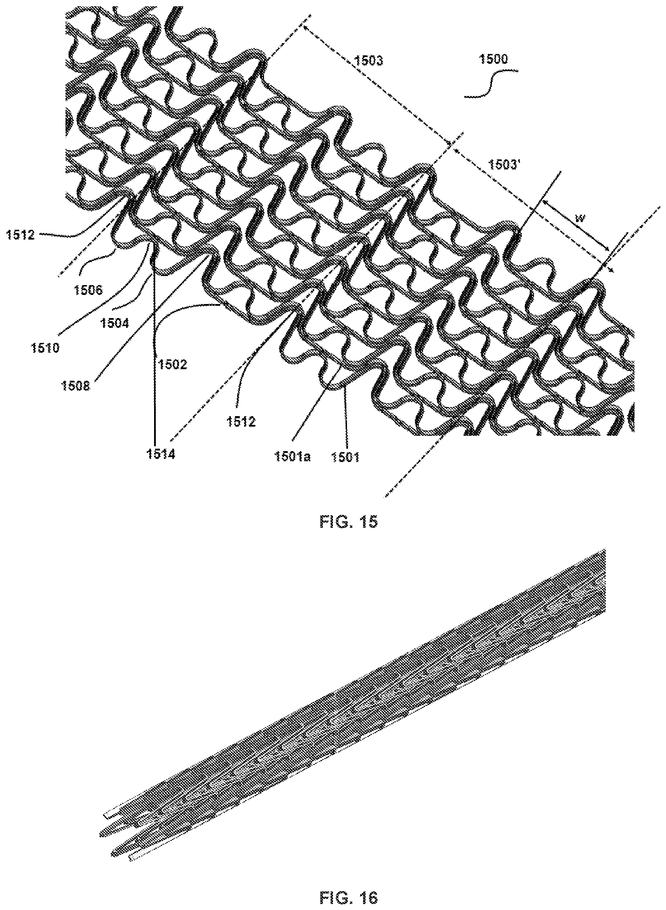

**FIGS. 12-15** show an aspect where the plurality of struts are arranged in a configuration wherein each strut of the plurality of struts is able to individually elongate in a circumferential direction along the length of the frame upon introduction of the medical device. For example, frame **1200** shows a plurality of struts, where the plurality of struts are arranged along a length of the frame **1200** in a plurality of parallel axially extending rows **1220.** An exemplary flattened schematic of the frame **1500** disclosed in these aspects is shown in **FIG. 15****.** Each row of the plurality of parallel axial rows can comprise an undulating pattern unit **1503.** In such exemplary aspects, each of the undulating pattern units **1503** can comprise a first valley **1502** having a substantially straight bottom portion, having a first width (w). The first valley, **1502,** is then followed by a first apex **1508,** a second valley **1504,** a second apex **1510,** and a third valley **1506.** In such exemplary configuration, a total width of the second **1504** and third valleys **1506** and the second apex **1510** is substantially the same as the first width (w) of the substantially straight bottom portion of the first valley **1502.**

In still further aspects, the third valley **1506** present in the frame **1500,** as shown in **FIG. 15****,** can be followed by a third apex **1512.** In further aspects, the third apex **1512** can be followed by a first valley **1502** of a next undulating pattern unit in the same row. It is understood that in certain and unlimiting aspects, each of the valleys or apexes in the same undulating unit **1503** and each subsequent undulating unit **1503'** can be formed from the same material. While in other unlimiting aspects, at least a portion of each of the valleys or apexes in the same undulating unit **1503** and each subsequent undulating unit **1503'** be formed from different materials or a combination of materials known in the art and applicable for the desired applications. In certain aspects, each component follows other components continuously without the presence of any connections or coupling elements between each component. However, in other exemplary and unlimiting aspects, at least a portion of the components of each of the undulating units **1503, 1503'** can be connected with a connector, or a coupling element, or a fastener. In such aspects, where the connectors, coupling elements, or fasteners are present, these elements can comprise any of such connectors, coupling elements, or fasteners known in the art and suitable for such purposed. Even further, it is understood that if any of those elements are present in the frame configuration, these elements can be made from any materials known in the art that can be used in the desired application.

In still further aspects, the plurality of parallel axial rows can be arranged such that a second apex **1510** in each repetitive pattern unit **1503** of each row is positioned above and is coupled with at least a portion of a substantially straight bottom portion of a first valley **1502** of an adjacent row by a bridging member **1514.** Bridging members **1214, 1314,** and **1414** can also be seen in **FIGS. 12-14****.** In still further aspects, the bridging member can be any member configured to connect or couple to elements of adjacent rows. In certain aspects, the bridging member **1214, 1314, 1414, 1514** can comprise a fastener. It is understood that in certain aspects, the fastener can comprise an adhesive material, such as, for example, a glue, or it can be a mechanical fastener, such as a weld, etc. It is understood that any materials that can be applicable for the desired application can be utilized as a fastener.

It is understood that each strut of the plurality of struts described in these aspects is configured to individually elongate. The configuration disclosed in these exemplary aspects allows the struts to move between elongated and shortened configurations at the longitudinal sections between bridging members.

It is also understood that in the disclosed configuration, the bridging member **1214, 1314, 1414, 1514** can assist in providing a desired expanded configuration of the frame.

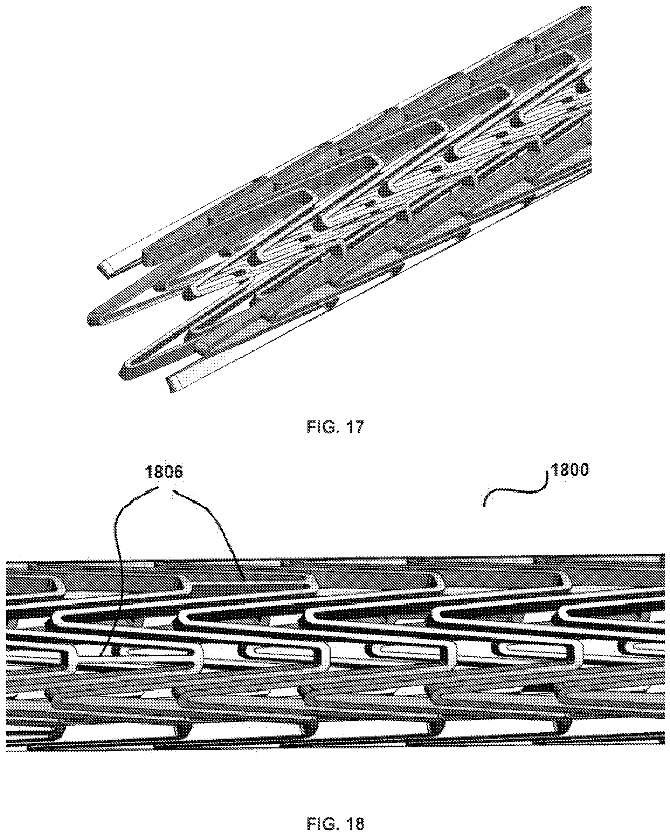

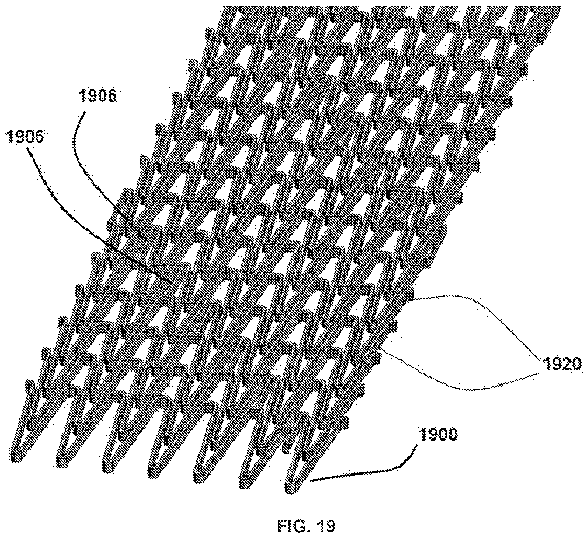

**FIG. 16** shows an additional aspect of the disclosed frame, while **FIGS. 17** and **18** show a magnified view of the distal end of the frame and the middle portion of the frame, respectively. **FIG. 19** shows a flattened view of the frame in the same configuration. It can be seen that in this exemplary configuration, the plurality of struts are arranged in a zig-zag configuration to form a plurality of parallel circumferential rows **1920,** wherein each row of the plurality of parallel circumferential rows **1920** comprises a plurality of straight portions circumferentially followed by a plurality of substantially bent portions; wherein adjacent rows of the plurality of parallel circumferential rows are coupled together by a singular bridging member **(1806** or **1906)** extending from a substantially bent portion in a first row **1920** to a corresponding substantially bent portion in an adjacent second row **1920.** In such exemplary aspects, the bridging member **1806, 1906** coupling any two adjacent rows is circumferentially offset from a bridging member coupling any following two adjacent rows. The offset between the vertical bridging members **1806, 1906** can enable the frame to substantially bent in any direction without kinking. It is understood that the bridging member **1806, 1906** can be made from any material that is applicable for the desired application. It is further understood that the bridging member **1806, 1906** can be provided by any known in the art methods. For example, the bridging member **1806, 1906** can be added after the frame is formed by welding or glue. While in yet other aspects, the bridging member is formed simultaneously with the plurality of struts. In certain aspects and similarly to other zig-zag configurations, each substantially straight portion of the plurality of straight portions can have equal lengths. While in other aspects, the plurality of straight portions can comprise one or more portions having unequal lengths at the distal and/or proximal ends of the frame.

Also, in some aspects, similarly to other zig-zag configurations disclosed herein, the plurality of substantially bent portions disclosed in this aspect can define a bisecting line extending through an apex of each substantially bent portion of the plurality of substantially bent portions, the bisecting line extending longitudinally along a length of the sheath, and wherein each substantially straight portion of the plurality of substantially straight portions has a first angle from the bisecting line from 0° to about 90°, including exemplary angles of about 5°, about 10°, about 15°, about 20°, about 25°, about 30 °, about 35°, about 40°, about 45°, about 50°, about 55°, about 60°, about 65°, about 70°, about 75°, about 80°, and about 85°. In yet further aspects, it is understood that when the frame is in the expanded position, the first angle increases during the expansion by greater than 0% to about 1,000% of its original value, including exemplary value of about 10%, about 20%, about 30%, about 40%, about 50%, about 60%, about 70%, about 80%, about 90%, about 100%, about 150%, about 200%, about 250%, about 300%, about 350%, about 400%, about 450%, about 500%, about 550%, about 600%, about 650%, about 700%, about 750%, about 800%, about 850%, about 900%, and about 950%. In yet further aspects, it is understood that the first angle can be increased by any value between any two foregoing values, for example, the first angle can be increased by greater than 0% to about 600% of its original value, or by 10% to about 400%, or by 100% to about 800%.

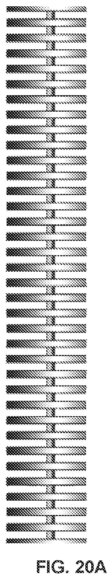

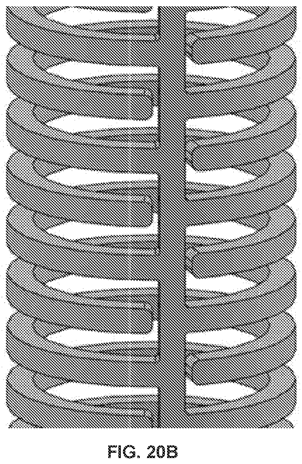

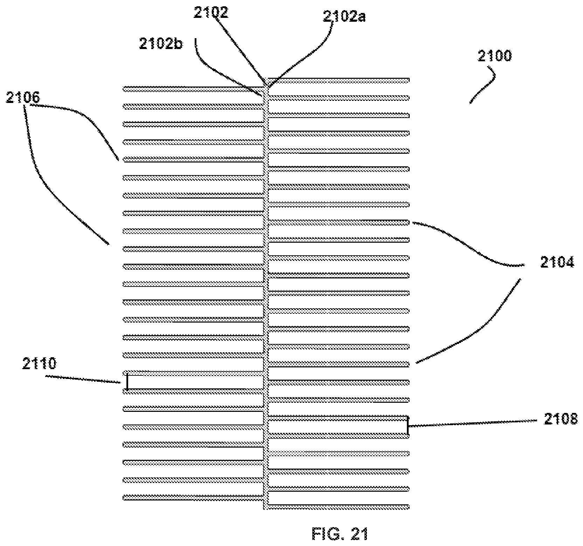

**FIGS. 20-26** show a frame configuration in other aspects. **FIG. 20A** shows a front view of this exemplary frame, **FIG. 20B** shows a back perspective view of the frame **2100,** and **FIG. 21** shows a flattened view of the frame **2100.** In this exemplary aspect, the cylindrical frame **2100** is formed by a first plurality of struts **2104** arranged along a first edge **2102a** of a singular vertical member **2102** and a second plurality of struts **2106** arranged along an opposite second edge **2102b** of the singular vertical member **2102.** The first and second plurality of struts **2104, 2106** curve around the longitudinal axis of the frame forming a central lumen therethrough. In still further aspects, each strut of the first plurality of struts **2104** is spaced from each other such that a first gap **2108** is formed between two adjacent struts in the first plurality of struts **2104.** While in other aspects, each strut of the second plurality of struts **2106** is spaced from each other such that a second gap **2110** is formed between two adjacent struts in the second plurality of struts **2106.** As can be seen in **FIG. 21****,** each strut of the first plurality of struts **2104** can be axially offset along the singular vertical member **2102** from each strut of the second plurality of struts **2106.** It is understood that when this exemplary frame is present in the unexpanded position, each strut of the first plurality of struts **2104** can be at least partially disposed within the second gap **2110,** and each strut of the second plurality of struts can be at least partially disposed within the first gap **2108.**

It is understood that the vertical member can have a predetermined width. In such aspects, a width of the vertical member can be less than 25% of a circumference of the cylindrical frame in the unexpanded position, or less than 20% of a circumference of the cylindrical frame in the unexpanded position, or less than 15% of a circumference of the cylindrical frame in the unexpanded position, or less than 10% of a circumference of the cylindrical frame in the unexpanded position, or less than 5 of a circumference of the cylindrical frame in the unexpanded position. It is understood that one of ordinary skills in the art can determine the circumference of the cylindrical frame in the unexpanded position based on d₁ of the frame. In still further aspects, the width of the vertical member can be measured along a portion of the circumference of the cylindrical frame corresponding to the vertical member.

Also disclosed herein are aspects where a width of the vertical member is less than about 20% of a length of at least one strut of the first and second plurality of struts; less than about 15% of a length of at least one strut of the first and second plurality of struts; less than about 10% of a length of at least one strut of the first and second plurality of struts; or less than about 5% of a length of at least one strut of the first and second plurality of struts. In still further aspects, the length of at least one strut of the first and second plurality of struts is measured between a proximal end of the corresponding strut adjacent the vertical member, and a distal end of the corresponding strut.

Also disclosed are aspects where a width of each strut of the first and second plurality of struts is less than about 25% of a circumference of the cylindrical frame in the unexpanded position; or less than about 20% of a circumference of the cylindrical frame in the unexpanded position; less than about 15% of a circumference of the cylindrical frame in the unexpanded position; less than about 10% of a circumference of the cylindrical frame in the unexpanded position; less than about 5% of a circumference of the cylindrical frame in the unexpanded position. In yet further aspects, the width of each strut of the first and second plurality of struts is measured in an axial direction along a longitudinal axis of the cylindrical frame.

Also disclosed are the aspects where a width of each strut of the first and second plurality of struts corresponds to a width of the vertical member. In yet further aspects, a width of each strut of the first and second plurality of struts is less than a width of the vertical member. In still further aspects, a width of each strut of the first and second plurality of struts is greater than a width of the vertical member.

Also disclosed are the aspects where a thickness of each strut of the first and second plurality of struts corresponds to a thickness of the vertical member. In yet other aspects, wherein a thickness of each strut of the first and second plurality of struts is greater than a thickness of the vertical member. While still in other aspects, a thickness of each strut of the first and second plurality of struts is less than a thickness of the vertical member.

In still further aspects, the struts' width can be anywhere from about 0.1 mm to about 3 mm, including exemplary values of about 0.2 mm, about 0.3 mm, about 0.4 mm, about 0.5 mm, about 0.6 mm, about 0.7 mm, about 0.8 mm, about 0.9 mm, about 1.0 mm, about 1.1 mm, about 1.2 mm, about 1.3 mm, about 1.4 mm, about 1.5 mm, about 1.6 mm, about 1.7 mm, about 1.8 mm, about 1.9 mm, about 2.0 mm, about 2.1 mm, 2.2 mm, about 2.3 mm, about 2.4 mm, about 2.5 mm, about 2.6 mm, about 2.7 mm, about 2.8 mm, and about 2.9 mm. It is understood that the struts can have any width value between any two foregoing values. For example and without limitation, the struts' width can be from about 0.2 mm to about 2.5 mm, or from about 0.5 mm to about 1.5 mm, or from about 1 mm to about 3 mm.

In still further aspects, the vertical member described herein can have the same width as the struts. However, in other aspects, the vertical member has a different width from the first plurality and the second plurality of the struts. In yet still further aspects, the vertical member can have a width anywhere from about 0.1 mm to about 3 mm, including exemplary values of about 0.2 mm, about 0.3 mm, about 0.4 mm, about 0.5 mm, about 0.6 mm, about 0.7 mm, about 0.8 mm, about 0.9 mm, about 1.0 mm, about 1.1 mm, about 1.2 mm, about 1.3 mm, about 1.4 mm, about 1.5 mm, about 1.6 mm, about 1.7 mm, about 1.8 mm, about 1.9 mm, about 2.0 mm, about 2.1 mm, 2.2 mm, about 2.3 mm, about 2.4 mm, about 2.5 mm, about 2.6 mm, about 2.7 mm, about 2.8 mm, and about 2.9 mm. It is understood that the medical member can have any width value between any two foregoing values. For example and without limitation, the vertical member's width can be from about 0.2 mm to about 2.5 mm, or from about 0.5 mm to about 1.5 mm, or from about 1 mm to about 3 mm.

In still further aspects, the first and/the second gap can be anywhere from about 0.1 mm to about 3 mm, including exemplary values of about 0.2 mm, about 0.3 mm, about 0.4 mm, about 0.5 mm, about 0.6 mm, about 0.7 mm, about 0.8 mm, about 0.9 mm, about 1.0 mm, about 1.1 mm, about 1.2 mm, about 1.3 mm, about 1.4 mm, about 1.5 mm, about 1.6 mm, about 1.7 mm, about 1.8 mm, about 1.9 mm, about 2.0 mm, about 2.1 mm, 2.2 mm, about 2.3 mm, about 2.4 mm, about 2.5 mm, about 2.6 mm, about 2.7 mm, about 2.8 mm, and about 2.9 mm. It is understood that the first and/or the second gap can have any value between any two foregoing values. For example, and without limitation, the first and the second gaps can be from about 0.2 mm to about 2.5 mm, or from about 0.5 mm to about 1.5 mm, or from about 1 mm to about 3 mm.

Also disclosed herein are the aspects where the thickness of the first and second plurality of struts is measured radially between in an inner surface and corresponding outer surface of each strut of the first and second plurality of struts, wherein the thickness of the vertical member is measured radially between an inner and outer surface of the vertical member. It is understood that any thickness can be used to make the struts, for example, from about 1 mil to about 200 mils, including exemplary values of about 2 mils, about 3 mils, about 5 mils, about 6 mils, about 7 mils, about 8 mils, about 9 mils, about 10 mils, about 20 mils, about 30 mils, about 40 mils, about 50 mils, about 60 mils, about 70 mils, about 90 mils, about 100 mils, about 120 mils, about 150 mils, and about 180 mils.

In yet further aspects, the thickness for the first and the second plurality of struts can be anywhere from about 0.1 mm to about 1.5 mm, including exemplary values of about 0.2 mm, about 0.3 mm, about 0.4 mm, about 0.5 mm, about 0.6 mm, about 0.7 mm, about 0.8 mm, about 0.9 mm, about 1.0 mm, about 1.1 mm, about 1.2 mm, about 1.3 mm, and about 1.4 mm. It is understood that the thickness of the first plurality of struts and the second plurality of struts can have any value between any two foregoing values. It is also understood that any struts described in any of the disclosed herein aspects can have a thickness that falls in the disclosed above range.

In yet further aspects, the d₂ of the frame in the expanded position is between 5 mm and 10 mm, including exemplary values of about 5.5 mm, about 6 mm, about 6.5 mm, about 7 mm, about 7.5 mm, about 8 mm, about 8.5 mm, about 9 mm, and about 9.5 mm. In yet further aspects, the d₂ of the frame in the expanded position is between 5 mm and 9 mm.

Still further disclosed are the aspects where a width of the first gap and/or a width of the second gap is greater than a width of each strut of the first and second plurality of struts.

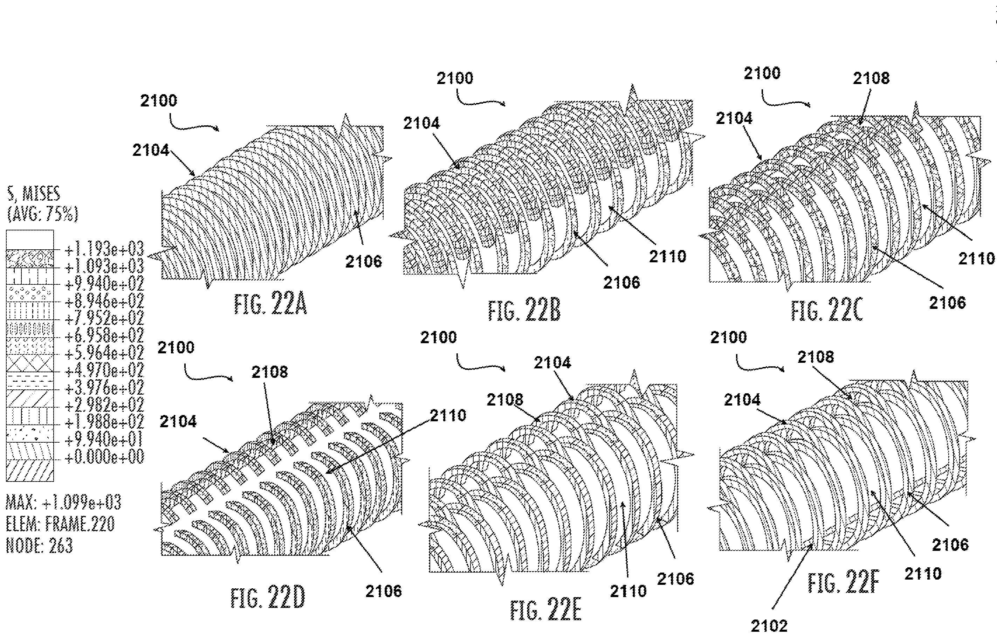

**FIGS. 22-26** show simulation of the stress generated on the disclosed frame when it passes from the unexpanded to expanded position due to the passage of the medical device through the central lumen of the frame. **FIGS. 22A-22F** show a front perspective view of the frame as it expands from the unexpanded position **FIG. 22A** to maximum stress applied in the expanded position in **FIG. 22D** and return to the unexpanded position in **FIG. 22E****.** As shown in **FIG. 22A****,** when the frame in the unexpanded position, the frame experiences no stress. The stress associated with the passage of medical devices increases, as shown in **FIGS. 22B-22D****,** with the greatest amount of stress is experienced along the middle length of the struts and the portion adjacent the vertical member **2102.**

As shown in **FIGS. 22B-22D****,** during expansion of the frame, the least amount of stress is experienced at the distal end of the struts (end opposite of the singular vertical member **2102).** As shown in **FIG. 22C****,** when the frame **2100** is fully expanded, the first and second plurality of struts **2104, 2106** are removed from their corresponding second and first gaps **2110, 2108,** and a space/gap is provided between the distal ends of the struts **2104, 2106.** That is, none of the first plurality of struts **2104** remains disposed within the second gap **2110,** and none of the second plurality of struts **2106** remains disposed within the first gap **2108.** In this configuration, the greatest amount of stress is experienced along the middle length of the strut and the portion adjacent the ventral member **2102,** and the least amount of stress is experienced at the distal end of the struts. After the passage of a medical device, the frame contracts and the stress is reduced **(****FIGS. 22E, 22F****).** As discussed in detail above, the frame can contract to a diameter that is substantially the same as the first diameter d₁ upon removal of the medical device, or it can contract to a diameter that is from 10% to 90% larger than the first diameter d₁ upon removal of the medical device. As discussed in detail above, the degree of frame contraction upon removal of the medical device can be designed by one of ordinary skills in the art depending on the size of the medical device and the possibility of hemorrhage if the frame contracts too much. **FIG. 22F** shows an aspect where a dimeter to which the frame contracts after simulated removal of the medical device is slightly larger than the original diameter, as shown in **FIG. 22A****.**

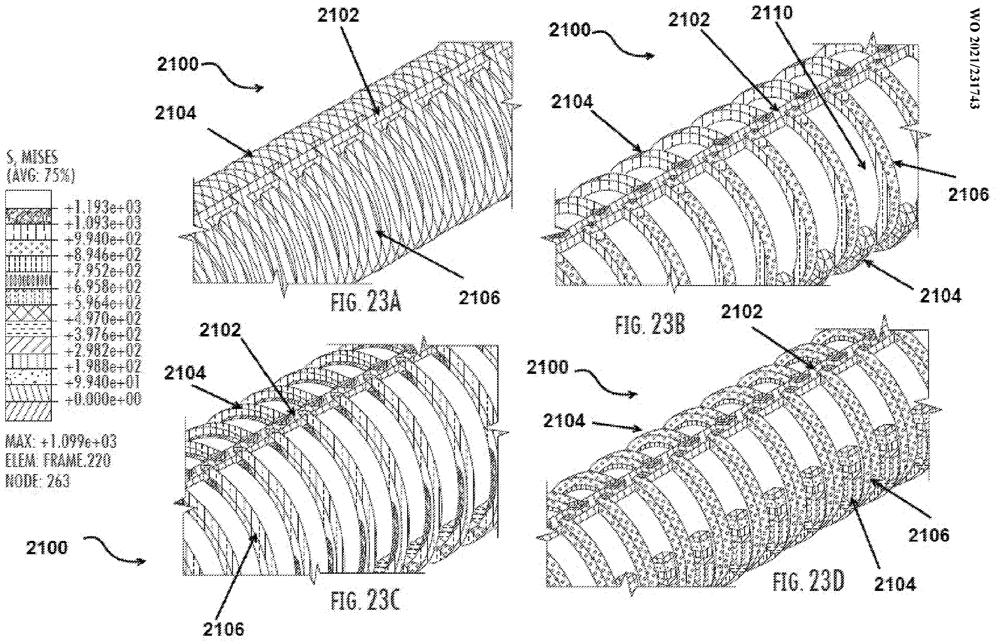

**FIGS. 23-23D** show a back perspective view of the frame as it expands from an unexpanded position **FIG. 23A** to the expanded position in **FIG. 23C** and returns towards an unexpanded position in **FIG. 23D****.** As illustrated in **FIG. 23A****,** the frame is in an unexpanded position and the least stress is experienced by the struts and the vertical member **2102.** As the frame **2100** expands **(****FIGS. 23B-23C****),** increased stress is experienced along the first and second plurality of struts **2104, 2016.** Increase stress is also experienced along the vertical member **2102** at the coupling between the vertical member **2102** and the struts **2104, 2106.** As provided in **FIGS. 23B-23D****,** the stress radiates out from the connection point between the struts **2104, 2106,** and the vertical member **2102,** with the most stress experienced adjacent the coupling point and reducing as it radiates outwards along the vertical member **2012.** As shown in **FIGS. 23B-23D****,** the stress at the coupling between the vertical member **2102** and the struts 2104, 2106 decreases as the struts return toward a non-expanded positioned (FIG. 23D).

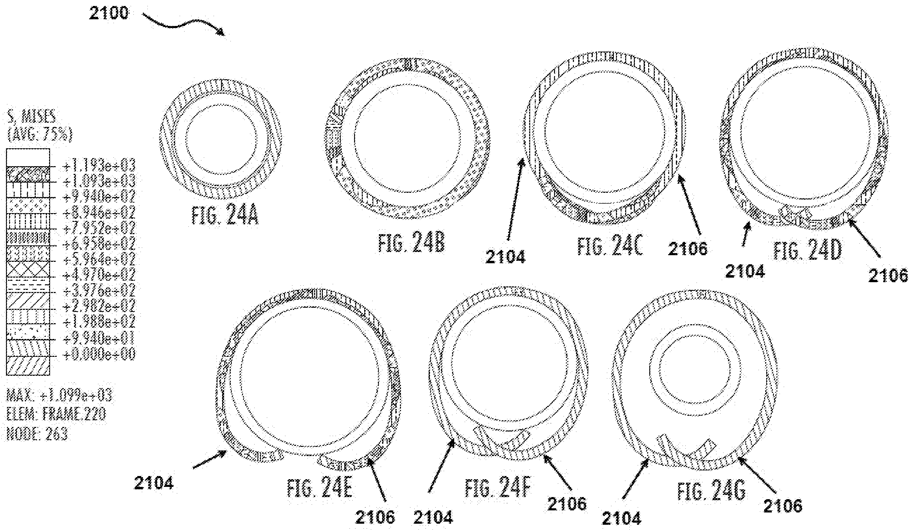

**FIGS. 24A-24G** show a cross-sectional/end view of the simulated stress results for the exemplary frame disclosed in **FIGS. 20-21****.** It can be seen that in these exemplary aspects, as the frame **2100** expands **(****FIGS. 24B-24D****),** the first plurality of struts **2104** remains disposed in the second gap **2110,** and the second plurality of struts **2106** remain disposed in the first gap **2108.** When the frame **2100** is fully expanded **(****FIG. 24E****),** the struts **2104, 2106** are removed from their corresponding gaps **2108, 2110,** and a space is provided between the distal ends of the struts **2104, 2106.** That is, none of the first plurality of struts **2104** remains disposed within the second gap **2110,** and none of the second plurality of struts **2106** remains disposed within the first gap **2108.** As illustrated in **FIGS. 24B-24E****,** during expansion, the greatest amount of stress is experienced along the middle length of the struts and the portion adjacent the vertical member **2102,** and the least amount of stress is experienced at the distal end of the struts (end opposite of the singular vertical member **2102).** It can also be seen that the dimeter to which the frame **2100** contracts after simulated removal of the medical device is significantly larger (FIG. 24G) than the original diameter, as shown in **FIG. 24A****.**

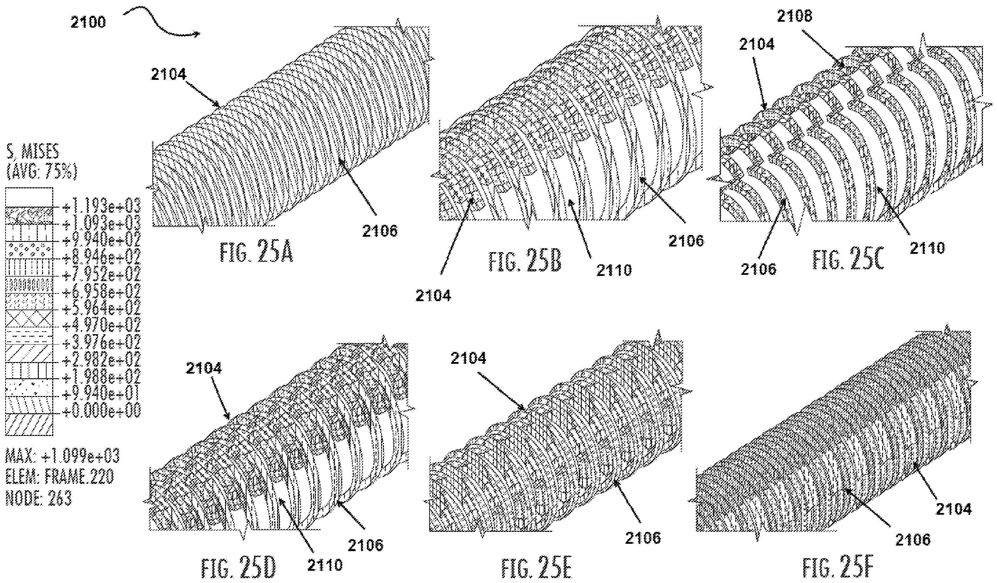

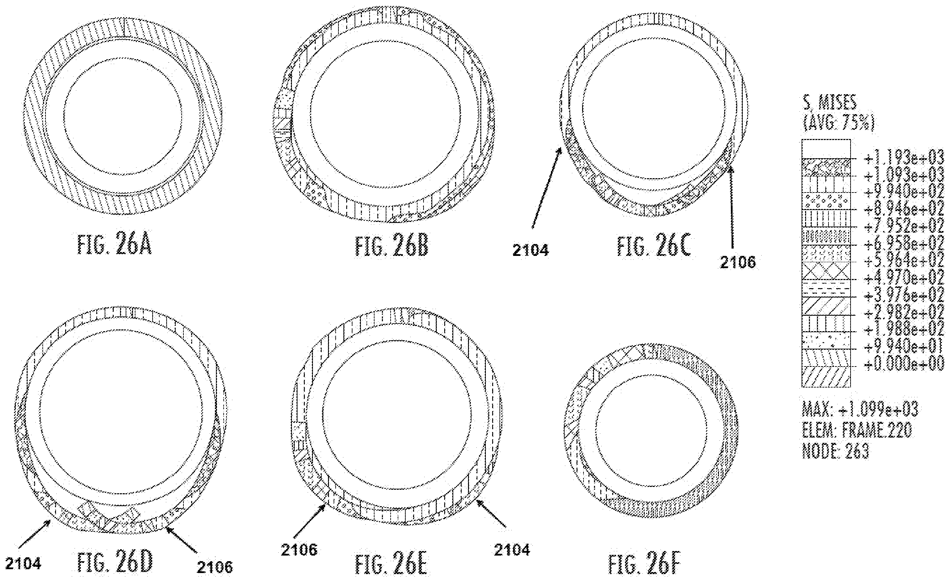

**FIGS. 25-26** show side perspective and cross section/end views of the simulated results for the exemplary frame **2100** as disclosed in **FIGS. 20-21****.** It can be seen that during the simulated passage of the medical device in this aspect when the frame **2100** is in the expanded position, at least a portion of the first plurality of struts **2104** remains at least partially disposed within the second gap **2110** and at least a portion of the second plurality of struts **2106** remains at least partially disposed within the first gap **2018 (****FIGS. 25B-25E** and **FIGS. 26B-26E****).** That is, unlike the simulation in **FIGS. 22-24****,** the first and second plurality of struts **2104, 2106** remain in the second and first gaps **2110, 2108,** respectively, when the frame is fully expanded **(****FIG. 25C** and **FIG. 26D****).** It can be further seen that upon removal of the simulated medical device, the frame **2100** contracts to a diameter that is substantially the same as the frame's diameter in the unexpanded position (compare **FIG. 25F** and original unexpanded frame of **FIG. 25A****,** see also **FIG. 26A** compared to **FIG. 26G).**

As shown in **FIG. 25B** and **FIGS. 26B-C****,** as the frame **2100** is expanding, the greatest amount of stress is experienced along the middle length of the struts and the portion adjacent the vertical member **2102,** and the least amount of stress is experienced at the distal end of the struts (the end opposite the vertical member **2102).** Similarly, when the frame **2100** is fully expanded **(****FIGS. 25C****,** **26D****),** the greatest amount of stress is experienced along the middle length of the strut and the portion adjacent the ventral member **2102,** and the least amount of stress is experienced at the distal end of the struts. As provided in **FIGS. 25D-25E** and **FIG. 26E****,** a similar stress pattern is experienced as the frame **2100** returns to an unexpanded configuration. **FIGS. 25F** and **26G** illustrate the frame **2100** returned to its final unexpanded configuration. As described above, upon removal of the medical device, the frame **2100** contracts to a diameter that is substantially the same as the frame's diameter in the unexpanded position. However, as illustrated in **FIGS. 25F** and **26G,** the frame **2100** experiences slightly increased stress in the final unexpanded configuration than in the original unexpanded configuration **(****FIGS. 25A****,** **26A****).**

As described above, the plurality of struts can be of any suitable material, including, for example, stainless steel, cobalt-chrome alloys, polymer, or any combination thereof. In certain exemplary and unlimiting aspects, the plurality of struts can be made of a shape memory material such as a nickel-titanium alloy knows as Nitinol that allows the frame to expand and contract to substantially original diameter if needed. In yet other exemplary aspects, when the frame is designed to contract to a diameter of about 10% to about 90% larger than the original diameter, a ductile material, such as stainless steel, can be used. However, it is understood that also nitinol can be used in the aspects where the frame is designed to contract to a diameter of about 10% to about 90% larger than the original diameter. In yet further aspects, the plurality of struts can comprise a nitinol, stainless steel, cobalt-chrome alloys, polymer, or any combination thereof. It is understood that if the polymer is chosen as a material for a plurality of struts, such polymer is a medical-grade polymer having the desired elastic properties. In yet further aspects, at least a portion of the disclosed herein frame exhibits an elastic deformation.

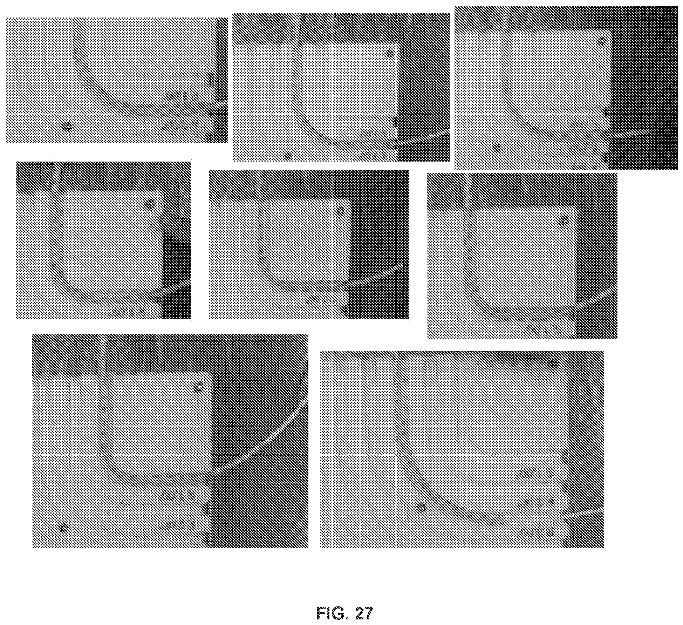

**FIG. 27** shows a photograph of an exemplary sheath comprising the disclosed herein expandable cylindrical frame as it curves while it passes through various conduits while maintaining kink resistance. It can be seen that the expandable cylindrical frame as it passes through a curved conduit can bend to form a curved portion having a radius corresponding to the curvature of the conduit. For example, the cylindrical frame can form a curve having a radius from about 1 inch to about 3 inches, including exemplary values of about 1.2 inches, about 1.5 inches, about 1.7 inches, about 2 inches, about 2.2 inches, about 2.5 inches, and about 2.7 inches without exhibiting any kinks.

In still further aspects, the sheath disclosed herein exhibits a decrease in an insertion force by greater than 0% to about 100%, including exemplary values of about 5%, about 10%, about 20%, about 30%, about 40%, about 50%, about 60%, about 70%, about 80%, and about 90%. It is understood that the decrease in the insertion force can have any percentage value between any two foregoing values.

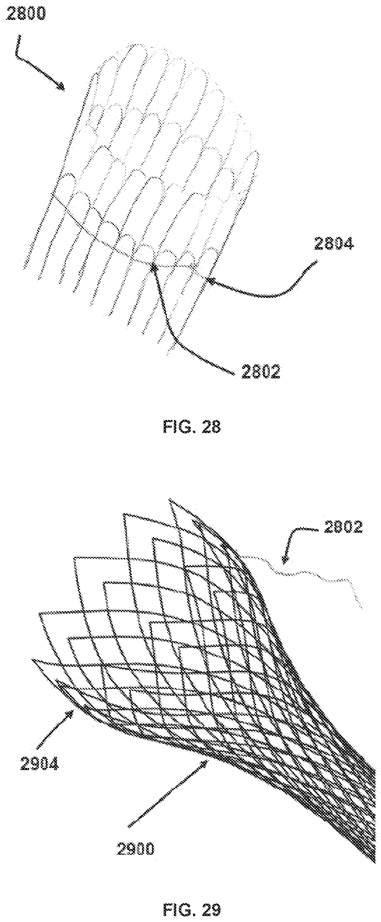

In still further aspects, the distal end of the frame **2800** can be modified to further facilitate the passage of the medical device **(****FIGS. 28** and **29****).** In such aspects, the distal end of the frame **2800** can comprise a pre-shaped flare **(****FIG. 29****)** that is configured to stay in a compressed state at the unexpanded position and to assume a flared configuration upon passing the medical device. It is understood that in such aspects, the pre-shaped flare can be kept in a compressed state by any means. In certain exemplary aspects, the pre-shaped flare can be kept in the compressed state by a filament **2802** attached to one or more of the plurality of struts **2804** at the distal end of the frame **2800.** The filament **2802** can extend circumferentially around the plurality of struts **2804** at the distal end 2900 of the frame **2800.** For example, the filament **2802** can extend circumferentially around all or a portion of the circumference of the distal end **2900** of the frame **2800.** It is understood that the term "filament" as referred in this aspect is not limiting to one filament and can, in fact, comprise a plurality of filaments, a string, or a suture.

It is further understood that the filament can be removably or fixedly attached to one or more of the plurality of struts **(2804,** **FIG. 28****)** by any methods known in the art. In certain aspects, it can be sutured to one or more struts. While in other aspects, it can be glued or tied to one or more struts. In certain aspects, the filament **2802** can be attached to any portion of the struts of any frame configuration disclosed herein. In yet further aspects, when the medical device passes through the distal end **2900** of the sheath, as shown in **FIG. 29****,** the filament **2802** is configured to tear away from the frame **2800** while still comprising at least one point of attachment with the one or more struts **2804,** thereby allowing the distal end **2900** of the frame **2800** to assume a flared configuration **2904** and increase the diameter of the opening at the distal end of the sheath. It is understood that maintaining at least one point of attachment with the struts **2804** prevents the filament **2802** from being released to the bloodstream upon tearing thereof. It is understood that in such aspects, the distal end **2900** can be heat set to flare or expand radially outwardly. In yet further aspects, the outward flare at the distal end **2900** can be provided with sufficient flexibility that the force exerted thereby on the surrounding arterial wall does not damage the tissue.

In still further aspects and as described here, the sheath can comprise one or more polymeric layers. In certain aspects, the one or more polymeric layers can comprise one or more of polyethylene, polypropylene, polyether block amide (PEBAX^{®}), silicon, or any combination thereof. In yet further aspects, at least a portion of the frame is embedded within the one or more polymeric layers. It is understood that the polymeric layers can comprise two or more layers. In certain aspects, the frame can be positioned between two or more polymeric layers. In the aspects where two or more polymeric layers are present, these polymeric layers can comprise the same or different material.

In certain aspects, a polymeric layer can be positioned such that it surrounds the frame as one cylindrical layer. It is understood that in such an aspect, at least a portion of the frame can be embedded within the layer. While in other aspects, the polymeric layer can be adjacent to an outer surface of the frame without at least a portion of the frame being embedded within it. In still further aspects, the sheath can comprise at least two polymeric layers that are positioned such that one polymeric layer is adjacent to an outer surface of the frame, and another polymeric layer is adjacent to an inner surface of the frame. In certain aspects, the frame can be at partially embedded in one of those polymeric layers. While in other aspects, the frame can be at least partially embedded in two of those exemplary polymeric layers. In yet still further aspects, the disclosed two exemplary polymeric layers can be in at least partial contact with each other.

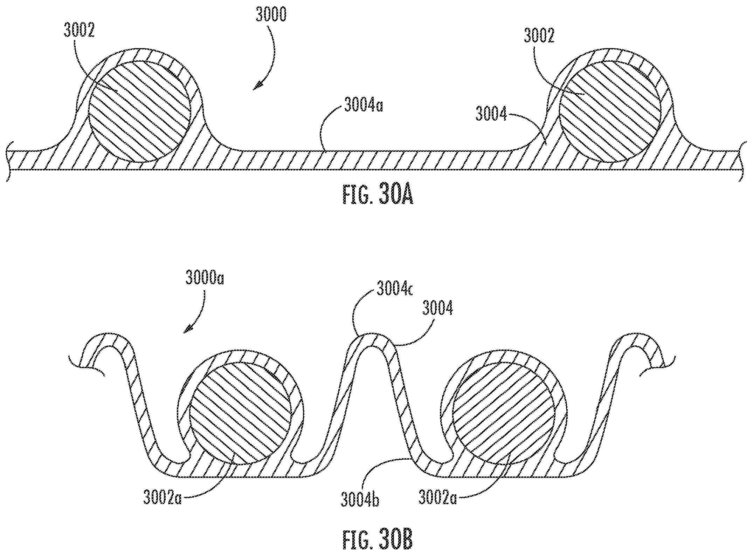

In still further aspects, the one or more polymeric layers can include a plurality of longitudinally-extending folds that extend circumferentially around the diameter of the sheath when the sheath is at the first diameter d₁. The longitudinally extending folds create a plurality of circumferentially spaced ridges and a plurality of circumferentially spaced valleys. As a medical device is passed through the sheath, the ridges and valleys level out to allow the sheath to radially expand. In yet further aspects, the one or more polymeric layers have a first thickness wherein the sheath at the first diameter d₁. In such exemplary aspects, as the medical device passes through the sheath, the one or more polymeric layers are thinning out to a second thickness to allow the sheath to radially expand. An exemplary aspect is shown in **FIGS. 30A-30B. FIG. 30A** illustrates a cross-sectional view of the sheath **3000a** along its longitudinal axis. The sheath can have any of the frame configurations disclosed herein. For example, the sheath **3000a** can have an exemplary frame configuration similar to those shown in **FIGS 20-26****.** **FIG. 30A** shows the exemplary rows **3002a** of the frame in the unexpanded position and surrounded by an exemplary polymeric layer **3004.** In the unexpanded position **(****FIG. 30A****),** the polymeric layer has a plurality of circumferentially spaced ridges **3004c** and a plurality of circumferentially spaced valleys **3004b** positioned between each row of the struts of the frame. When sheath **3000** expands **(****FIG.30B****),** the ridges and valleys level out to form an extended polymeric layer **3004a** and **3000** around the embedded rows **3002** of the frame. When the sheath is back to the unexpended position **(****FIG. 30A****),** the ridges and valleys are formed again.

In still further aspects, the plurality of struts of the disclosed frame configurations can be movable between one or more polymeric layers such that the plurality of struts can radially expand as a medical device is passed through the sheath while the length of the sheath remains substantially constant, and, in some aspects, the plurality of struts are not engaged or adhered to one or more polymeric layers at all.

In certain aspects, the one or more polymeric layers can comprise a relatively thin layer of polymeric material. For example, in some aspects, the thickness of the one or more polymeric layers can be from 0.01 mm to 0.5 mm, including exemplary values of about 0.05 mm, about 0.1 mm, about 0.15 mm, about 0.2 mm, about 0.25 mm, about 0.3 mm, about 0.35 mm, about 0.4 mm, and about 0.45 mm.

In certain examples, the one or more polymeric layers can comprise a lubricious, low-friction, and/or relatively non-elastic material. In particular aspects, the one or more elastomeric materials can comprise a polymeric material having a modulus of elasticity of 400 MPa or greater. Exemplary materials can include ultrahigh-molecular-weight polyethylene (UHMWPE) (e.g., Dyneema^{®}), high-molecular-weight polyethylene (HMWPE), or polyether ether ketone (PEEK). In still further aspects, the materials can also comprise low-density polyethylene (LDPE), bi-oriented polypropylene, cast polypropylene, thermoplastic polyurethane, and/or combinations of any of the above. It is understood that such a low coefficient of friction materials can facilitate passage of the prosthetic device through the lumen of the sheath. Other suitable materials for the one or more polymeric layers can include polytetrafluoroethylene (PTFE), expanded polytetrafluoroethylene (ePTFE), ethylene tetrafluoroethylene (ETFE), nylon, polyethylene, polyether block amide (e.g., Pebax), and/or combinations of any of the above. Some aspects of the disclosed herein sheath can include a lubricious liner on an inner surface of the one or more polymeric layers surrounding the frame. Examples of suitable lubricious liners include materials that can further reduce the coefficient of friction, such as PTFE, polyethylene (such as, for example, HMWPE, UHMWPE, LDPE, HDPE), polyvinylidine fluoride, and combinations thereof. Suitable materials for a lubricious liner also include other materials desirably having a coefficient of friction of 0.1 or less.

In yet further aspects, the sheath disclosed herein can comprise an additional outer layer. In such exemplary aspects, the outer layer can be adjacent to one or more polymeric layers surrounding the frame. In such aspects, the outer layer can be an additional resilient, elastic layer. In certain aspects, the outer layer can be configured to apply force to the one or more polymeric layers in a radial direction when the sheath expands beyond its natural diameter by passage of the delivery apparatus through the sheath. Stated differently, the outer layer can be configured to apply encircling pressure to one or more polymeric layers, and the frame beneath the outer layer counteracts expansion of the sheath. The radially inwardly directed force is sufficient to cause the sheath to collapse radially back to its unexpanded state after the delivery apparatus is passed through the sheath. In still further aspects, the outer layer, as disclosed herein, can also take other forms, such as a tubular layer comprising an elastomeric material, a mesh, a shrinkable polymer layer such as a heat-shrink tubing layer, etc. In lieu of, or in addition to, the outer layer, the disclosed herein sheath can also include an elastomeric or heat-shrink tubing layer around the one or more polymeric layers. Examples of such elastomeric layers are disclosed in U.S. Publication No. 2014/0379067, U.S. Publication No. 2016/0296730, and U.S. Publication No. 2018/0008407.

In still further aspects, the disclosed herein sheath can comprise an exterior hydrophilic coating on an outer surface of the one or more polymeric layers. Such a hydrophilic coating can facilitate the insertion of the sheath into a patient's vessel, reducing potential damage. Examples of suitable hydrophilic coatings include the Harmony^{™} Advanced Lubricity Coatings and other Advanced Hydrophilic Coatings available from SurModics, Inc., Eden Prairie, MN. DSM medical coatings (available from Koninklijke DSM N.V, Heerlen, the Netherlands), as well as other hydrophilic coatings (*e.g.,* PTFE, polyethylene, polyvinylidene fluoride). Such hydrophilic coatings may also be included on an inner surface of the one or more polymeric layers to reduce friction between the sheath and the delivery system, thereby facilitating the use and improving safety. In some aspects, a hydrophobic coating, such as Perylene, can be used in order to reduce friction.

Various examples of polymeric layers and their configuration in the sheath can be found in U.S. Patent Application Nos. 62,912,569, 16/378,417, and 15/875,706.

### METHODS

Turning out to the methods of making the expandable sheath disclosed herein. In certain aspects disclosed herein is a method comprising arranging at least one plurality of struts to form an expandable cylindrical frame having a proximal and distal end, an inner surface and an outer surface, and a central axis extending along a length of the frame; wherein the expandable cylindrical frame has a first length and an opposite second length, and wherein in a first position at least a portion of the first length and at least a portion of the second length are substantially the same; wherein the expandable cylindrical frame is movable from the first position to a second position by curving away from the central axis, wherein at the second position at least a portion of the first length shortens and at least a portion of the second length elongates; wherein the inner surface of the expandable cylindrical frame defines a lumen configured to receive a medical device; and wherein the expandable cylindrical frame is configured to expand from a first diameter d₁ in an unexpended position to a second diameter d₂ an expanded position upon passage of a medical device. It is understood that the methods disclosed herein include arranging that the plurality of struts such that any of the disclosed herein expandable cylindrical configurations can be formed.

It is further understood that the plurality of struts can be formed within the specific configuration by laser cutting desired patterns and arranging them in the desired configurations. In yet other aspects, the plurality of struts and configurations can be formed by any other known in the art methods. For example, and without limitation, such configurations can be formed by 3-D printing, molding, etc.

In still further aspects, the methods disclosed herein comprise forming a pre-shaped flare at the distal end of the frame such that the pre-shaped flare is configured to stay compressed at the rest position and to assume a flared configuration upon passing the medical device. In such exemplary aspects, the pre-shaped flare can be prepared by a heat-set of the distal portion of the plurality of the struts. In yet further aspects, the filament can be attached to the distal portion of the plurality of struts by any methods known in the art. For example, and without limitations, the filament can be attached to the plurality of struts by methods comprising gluing, tying, welding, suturing, or any combination thereof.

In yet further aspects, the methods disclosed herein comprise applying one or more polymeric layers to the expandable cylindric frame. It is understood that such layers can be applied by any methods known in the art. In some aspects, the frame can be sandwiched between two or more polymeric layers. Yet, in other aspects, the frame can be inserted in a cylindrical tubing comprising one or more disclosed herein polymeric layers. In yet further aspects, the frame can be dipped into a solution comprising the desired polymer to form the one or more polymeric layers. Yet, in still further aspects, the polymeric layers can be formed by wrapping at least one layer of the polymeric film around the frame. In yet still further aspects, two or more layers of the polymeric films can be wrapped around the frame. In yet still, further aspects, if the two or more layers of the polymeric film are present, these layers can comprise the same polymer or different polymers. In such exemplary and unlimiting aspects, a first polymer can be wrapped around the frame to form one or more layers, and then a second polymer can be outwardly positioned on the first polymer by wrapping or dipping into the polymer solution or by positioning a tube comprising the second polymer, and so on, and then reflowing all layers together to form a laminate material.

In some aspects, where application of heat and pressure is needed during the method of making the expandable sheath, it can be achieved by placing the mandrel in a vessel containing a thermally-expandable material and heating the thermally-expandable material in the vessel. In some aspects, a radial pressure of 100 MPa or more is applied to the mandrel via the thermally-expandable material. In some aspects, applying heat and pressure further comprises applying a heat shrink tubing layer over one or more polymeric layers and applying heat to the heat shrink tubing layer. Additionally, exemplary methods of forming the one or more layers can be found in U.S. Patent Application No. 16/378,417.

Although several aspects of the disclosure have been disclosed in the foregoing specification, it is understood by those skilled in the art that many modifications and other aspects of the disclosure will come to mind to which the disclosure pertains, having the benefit of the teaching presented in the foregoing description and associated drawings. It is thus understood that the disclosure is not limited to the specific aspects disclosed hereinabove and that many modifications and other aspects are intended to be included within the scope of the appended claims. Moreover, although specific terms are employed herein, as well as in the claims which follow, they are used only in a generic and descriptive sense and not for the purposes of limiting the described disclosure nor the claims which follow.

## Claims

1. An expandable sheath (100; 300) for deploying a medical device (10, 12) comprising:
at least one plurality of struts arranged to form an expandable cylindrical frame (500; 600; 800; 900; 1000; 1100; 1800; 1900) having a proximal and distal end, an inner surface and an outer surface, and a central axis (302) extending along a length of the frame (500; 600; 800; 900; 1000; 1100; 1800; 1900);
wherein the plurality of struts are arranged to form a zig-zag configuration comprising a plurality of substantially straight portions (502, 502a; 602, 602a; 802, 802a) circumferentially followed by a plurality of substantially bent portions, wherein each straight portion (502, 502a; 602, 602a; 802, 802a) is followed by a bent portion (504; 604; 818);
wherein the expandable cylindrical frame (500; 600; 800; 900; 1000; 1100; 1800; 1900) has a first length (304) and an opposite second length (306), and wherein, in a first position, at least a portion of the first length (304) and at least a portion of the second length (306) are substantially the same,
wherein the expandable cylindrical frame (500; 600; 800; 900; 1000; 1100; 1800; 1900) is movable from the first position to a second position by curving away from the central axis (302), wherein at the second position at least a portion of the first length (304') shortens and at least a portion of the second length (306') elongates;
wherein the inner surface of the expandable cylindrical frame (500; 600; 800; 900; 1000; 1100; 1800; 1900) defines a lumen configured to receive a medical device (10, 12); and
wherein the expandable cylindrical frame (500; 600; 800; 900; 1000; 1100; 1800; 1900) is configured to expand from a first diameter (d₁) in an unexpended position to a second diameter (d₂) in an expanded position upon passage of a medical device (10, 12); and wherein the expandable cylindrical frame (500; 600; 800; 900; 1000; 1100; 1800; 1900) is kink-resistant.

2. The expandable sheath (100; 300) of claim 1, wherein the portion of the first length and the portion of the second length (306') in the second position are located along a curved portion of the frame (500; 600; 800; 900; 1000; 1100; 1800; 1900), and/or
wherein, preferably, the first length (304, 304') is provided on a first portion of an outer diameter of the expandable cylindrical frame (500; 600; 800; 900; 1000; 1100; 1800; 1900) and the second length (306, 306') is provided on a second portion of the outer diameter of the expandable cylindrical frame (500; 600; 800; 900; 1000; 1100; 1800; 1900), wherein the first portion is circumferentially opposite to the second portion of the outer diameter, and/or
wherein, further preferably, the first diameter (d₁) is from about 3 mm to about 5 mm, and wherein the second diameter (d₂) is from about 5 mm to about 10 mm.

3. The expandable sheath (100; 300) of any one of claims 1 or 2, wherein the zig-zag configuration is wound along a central axis (302) to form the frame (500; 600), or
wherein the zig-zag configuration forms a plurality of parallel circumferential rows (816); and wherein the plurality of parallel circumferential rows (816) are axially coupled to each other by a singular vertical strut member (806; 906; 1006; 1106) disposed along a length of the frame (800; 900; 1000; 1100), or
wherein the zig-zag configuration forms a plurality of parallel circumferential rows (1920); wherein adjacent rows of the plurality of parallel circumferential rows(1920) are coupled together by a singular bridging member (1806; 1906) extending from a substantially bent portion in a first row to a corresponding substantially bent portion in an adjacent second row (1920), and wherein the singular bridging member (1806; 1906) coupling any two adjacent rows (1920) is circumferentially offset from a bridging member (1806; 1906) coupling any following two adjacent rows (1920).

4. The expandable sheath (100; 300) of claim 3, wherein each substantially straight portion (502, 502a; 602, 602a; 802, 802a) of the plurality of substantially straight portions (502, 502a; 602, 602a; 802, 802a) being of equal lengths.

5. The expandable sheath of claim 3, wherein the plurality of substantially straight portions (502, 502a; 602, 602a; 802, 802a) comprise one or more portions having unequal lengths at the distal and/or proximal ends of the frame.

6. The expandable sheath (100; 300) of any one of claims 3 to 5, wherein the plurality of substantially bent portions (504; 604; 818) define a bisecting line extending through an apex of the plurality of substantially bent portions (504; 604; 818) and extending longitudinally along a length of the sheath, and wherein each substantially straight portion (502, 502a; 602, 602a; 802, 802a) of the plurality of substantially straight portions (502, 502a; 602, 602a; 802, 802a) has a first angle from the bisecting line from 0° to about 90°.

7. The expandable sheath (100; 300) of claim 6, wherein the frame is in the expanded position, the first angle increases by from greater than 0% to about 600% of its original value.

8. The expandable sheath (100; 300) of any one of claims 3 to 7, wherein the singular vertical strut member (806; 906; 1006; 1106) extends longitudinally along a length of the sheath and connects apexes of the substantially bent portions of each row of the plurality of parallel circumferential rows, and the vertical strut member forms a second angle with each substantially straight portion of the corresponding adjacent substantially straight portions of 0° to about 90°, and/or
wherein, preferably, upon introduction of the medical device (10, 12), the frame (806; 906; 1006; 1106) expands circumferentially in a direction opposite to the single vertical strut member (806; 906; 1006; 1106), and wherein the frame (806; 906; 1006; 1106) does not expand along the singular vertical strut member (806; 906; 1006; 1106).

9. An expandable sheath (100, 300) for deploying a medical device (10, 12) comprising:
at least one plurality of struts arranged to form an expandable cylindrical frame (1200; 1300; 1400; 1500) having a proximal and distal end, an inner surface and an outer surface, and a central axis (302) extending along a length of the frame (1200; 1300; 1400; 1500);
wherein the expandable cylindrical frame (1200; 1300; 1400; 1500) has a first length (304) and an opposite second length (306), and wherein, in a first position, at least a portion of the first length (304) and at least a portion of the second length (304) are substantially the same,
wherein the expandable cylindrical frame (1200; 1300; 1400; 1500) is movable from the first position to a second position by curving away from the central axis (302), wherein at the second position at least a portion of the first length (304') shortens and at least a portion of the second length (306') elongates;
wherein the inner surface of the expandable cylindrical frame (1200; 1300; 1400; 1500) defines a lumen configured to receive a medical device (10, 12); and
wherein the expandable cylindrical frame (1200; 1300; 1400; 1500) is configured to expand from a first diameter (d₁) in an unexpended position to a second diameter (d₂) in an expanded position upon passage of a medical device (10, 12); and wherein the expandable cylindrical frame (1200; 1300; 1400; 1500) is kink-resistant,
wherein the plurality of struts are arranged in a configuration wherein each strut of the plurality of struts is able to individually elongate in a circumferential direction along the length of the frame (1200; 1300; 1400; 1500) upon introduction of the medical device (10, 12), and
wherein the plurality of struts are arranged in a plurality of parallel axial rows (1220), and wherein each row (1220) of the plurality of parallel axial rows (1220) comprises an undulating pattern unit (1503), wherein each unit (1503) comprises:
a first valley (1502) having a substantially straight bottom portion, wherein the substantially straight bottom portion of the first valley (1502) has a first width (w),
wherein the first valley (1502) is followed by a first apex (1508), a second valley (1504), a second apex (1510), and a third valley (1506), and
wherein a total width of the second and third valleys (1504, 1506) and the second apex (1510) is substantially the same as the first width (w) of the substantially straight bottom portion of the first valley (1502).

10. The expandable sheath (100; 300) of claim 9, wherein the third valley (1506) is followed by a third apex (1512) and a first valley (1502) of a next undulating pattern unit (1503) in the same row (1220), and/or
wherein, preferably, the plurality of parallel axial rows are arranged such that a second apex in each repetitive pattern unit of each row is positioned above and is coupled with at least a portion of a substantially straight bottom portion of a first valley of an adjacent row by a bridging member.

11. The expandable sheath (100; 300) of any one of claims 1 to 10, wherein the distal end of the frame (2800; 2900) comprises a pre-shaped flare that is configured to stay in a compressed state at the unexpanded position and to assume a flared configuration upon passing the medical device (10, 12),
wherein, preferably, the pre-shaped flare is kept in the compressed state by a filament (2802) attached to one or more of the plurality of struts (2804) at the distal end of the frame and extending circumferentially around the plurality of struts (2804) at the distal end, and
wherein, further preferably, the distal end of the frame (2800; 2900) is in the expanded position, the filament (2802) stays attached to one or more of the plurality of struts (2804) at the distal end of the frame but does not extends circumferentially around the plurality of struts (2804) at the distal end allowing the distal end of the frame (2800; 2900) to flare radially outward.

12. The expandable sheath (100; 300) of any one of claims 1 to 11, wherein the frame is configured to contract to a diameter that is from about 10% to about 90% larger than the first diameter (d₁) upon removal of the medical device (10, 12).

13. The expandable sheath (100; 300) of any one of claims 1 to 12, wherein the plurality of struts comprise a nitinol, stainless steel, cobalt-chrome alloys, polymer, or any combination thereof.

14. The expandable sheath (100; 300) of any one of claims 1 to 13, wherein the sheath further comprises one or more polymeric layers (3004; 3004a), wherein the one or more polymeric layers (3004; 3004a) comprise polyethylene, polypropylene, polyether block amide (PEBAX^{®}), silicon, or any combination thereof,
wherein, preferably, at least a portion of the frame is embedded within the one or more polymeric layers (3004; 3004a), and
wherein, further preferably, the one or more polymeric layers (3004; 3004a) comprise a plurality of longitudinally extending folds wherein the sheath is at the first diameter (d₁), and wherein the longitudinally extending folds create a plurality of circumferentially spaced ridges (3004c) and a plurality of circumferentially spaced valleys (3004b), and
wherein, preferably, as the medical device (10, 12) passes through the sheath, the ridges (3004c) and valleys (3004b) level out to allow the sheath to radially expand.

15. The expandable sheath (100; 300) of claim 14, wherein the one or more polymeric layers (3004; 3004a) have a first thickness wherein the sheath at the first diameter (d₁), and wherein, as the medical device (10, 12) passes through the sheath, the one or more polymeric layers (3004; 3004a) are thinning out to a second thickness to allow the sheath to radially expand.

## Patentansprüche

1. Expandierbare Hülle (100; 300) zum Ausbringen einer medizinischen Vorrichtung (10, 12), umfassend:
wenigstens eine Vielzahl von Streben, die so angeordnet sind, dass sie einen expandierbaren zylindrischen Rahmen (500; 600; 800; 900; 1000; 1100; 1800; 1900) bilden, der ein proximales und ein distales Ende, eine Innenfläche und eine Außenfläche und eine Mittelachse (302), die sich entlang einer Länge des Rahmens (500; 600; 800; 900; 1000; 1100; 1800; 1900) erstreckt, aufweist;
wobei die Vielzahl von Streben so angeordnet ist, dass sie eine Zick-Zack-Konfiguration bilden, die mehrere im Wesentlichen gerade Abschnitte (502, 502a; 602, 602a; 802, 802a) umfasst, auf die in Umfangsrichtung eine Vielzahl von im Wesentlichen gebogenen Abschnitten folgt, wobei auf jeden geraden Abschnitt (502, 502a; 602, 602a; 802, 802a) ein gebogener Abschnitt (504; 604; 818) folgt;
wobei der expandierbare zylindrische Rahmen (500; 600; 800; 900; 1000; 1100; 1800; 1900) eine erste Länge (304) und eine gegenüberliegende zweite Länge (306) aufweist, und wobei in einer ersten Stellung wenigstens ein Abschnitt der ersten Länge (304) und wenigstens ein Abschnitt der zweiten Länge (306) im Wesentlichen gleich sind,
wobei der expandierbare zylindrische Rahmen (500; 600; 800; 900; 1000; 1100; 1800; 1900) von der ersten Stellung in eine zweite Stellung bewegbar ist, indem er sich von der Mittelachse (302) weg krümmt, wobei sich in der zweiten Stellung wenigstens ein Abschnitt der ersten Länge (304') verkürzt und wenigstens ein Abschnitt der zweiten Länge (306') verlängert;
wobei die Innenfläche des expandierbaren zylindrischen Rahmens (500; 600; 800; 900; 1000; 1100; 1800; 1900) ein Lumen definiert, das zur Aufnahme einer medizinischen Vorrichtung (10, 12) konfiguriert ist; und
wobei der expandierbare zylindrische Rahmen (500; 600; 800; 900; 1000; 1100; 1800; 1900) dazu konfiguriert ist, beim Passieren einer medizinischen Vorrichtung (10, 12) von einem ersten Durchmesser (d₁) in einer nicht expandierten Stellung auf einen zweiten Durchmesser (d₂) in einer expandierten Stellung zu expandieren; und wobei der expandierbare zylindrische Rahmen (500; 600; 800; 900; 1000; 1100; 1800; 1900) knickfest ist.

2. Expandierbare Hülle (100; 300) nach Anspruch 1,
wobei der Abschnitt der ersten Länge und der Abschnitt der zweiten Länge (306') in der zweiten Stellung entlang eines gekrümmten Abschnitts des Rahmens (500; 600; 800; 900; 1000; 1100; 1800; 1900) angeordnet sind, und/oder
wobei vorzugsweise die erste Länge (304, 304') an einem ersten Abschnitt eines Außendurchmessers des expandierbaren zylindrischen Rahmens (500; 600; 800; 900; 1000; 1100; 1800; 1900) vorgesehen ist und die zweite Länge (306, 306') an einem zweiten Abschnitt des Außendurchmessers des expandierbaren zylindrischen Rahmens (500; 600; 800; 900; 1000; 1100; 1800; 1900) vorgesehen ist, wobei der erste Abschnitt in Umfangsrichtung gegenüber dem zweiten Abschnitt des Außendurchmessers liegt, und/oder
wobei, noch mehr bevorzugt, der erste Durchmesser (d₁) von etwa 3 mm bis etwa 5 mm beträgt und wobei der zweite Durchmesser (d₂) von etwa 5 mm bis etwa 10 mm beträgt.

3. Expandierbare Hülle (100; 300) nach einem der Ansprüche 1 oder 2,
wobei die Zick-Zack-Konfiguration entlang einer zentralen Achse (302) gewickelt ist, um den Rahmen (500; 600) zu bilden, oder
wobei die Zick-Zack-Konfiguration mehrere parallele Umfangsreihen (816) bildet; und wobei die mehreren parallelen Umfangsreihen (816) durch ein einzelnes vertikales Strebenelement (806; 906; 1006; 1106), das entlang einer Länge des Rahmens (800; 900; 1000; 1100) angeordnet ist, axial miteinander gekoppelt sind, oder
wobei die Zick-Zack Konfiguration mehrere parallele Umfangsreihen (1920) bildet; wobei benachbarte Reihen der mehreren parallelen Umfangsreihen (1920) durch ein einzelnes Brückenelement (1806; 1906), das sich von einem im Wesentlichen gebogenen Abschnitt in einer ersten Reihe zu einem entsprechenden im Wesentlichen gebogenen Abschnitt in einer benachbarten zweiten Reihe (1920) erstreckt, miteinander gekoppelt sind, und wobei das einzelne Brückenelement (1806; 1906), das irgendwelche zwei benachbarten Reihen (1920) koppelt, in Umfangsrichtung gegenüber einem Brückenelement (1806; 1906) versetzt ist, das irgendwelche folgenden zwei benachbarten Reihen (1920) koppelt.

4. Expandierbare Hülle (100; 300) nach Anspruch 3, wobei jeder im Wesentlichen gerade Abschnitt (502, 502a; 602, 602a; 802, 802a) der mehreren im Wesentlichen geraden Abschnitte (502, 502a; 602, 602a; 802, 802a) die gleiche Länge aufweist.

5. Expandierbare Hülle nach Anspruch 3, wobei die mehreren im Wesentlichen geraden Abschnitte (502, 502a; 602, 602a; 802, 802a) einen oder mehrere Abschnitte mit ungleichen Längen an den distalen und/oder proximalen Enden des Rahmens umfassen.

6. Expandierbare Hülle (100; 300) nach einem der Ansprüche 3 bis 5, wobei die mehreren im Wesentlichen gebogenen Abschnitte (504; 604; 818) eine Halbierungslinie definieren, die sich durch einen Scheitelpunkt der mehreren im Wesentlichen gebogenen Abschnitte (504; 604; 818) verläuft und sich in Längsrichtung entlang einer Länge der Hülle erstreckt, und wobei jeder im Wesentlichen gerade Abschnitt (502, 502a; 602, 602a; 802, 802a) der mehreren im Wesentlichen geraden Abschnitte (502, 502a; 602, 602a; 802, 802a) einen ersten Winkel von der Halbierungslinie von 0° bis etwa 90° aufweist.

7. Expandierbare Hülle (100; 300) nach Anspruch 6, wobei der Rahmen in der expandierten Stellung den ersten Winkel um mehr als 0% bis etwa 600% seines ursprünglichen Wertes vergrößert.

8. Expandierbare Hülle (100; 300) nach einem der Ansprüche 3 bis 7,
wobei sich das einzelne vertikale Strebenelement (806; 906; 1006; 1106) in Längsrichtung entlang einer Länge der Hülle erstreckt und die Scheitelpunkte der im Wesentlichen gebogenen Abschnitte jeder Reihe der mehreren parallelen Umfangsreihen verbindet, und das vertikale Strebenelement mit jedem im Wesentlichen geraden Abschnitt der entsprechenden benachbarten im Wesentlichen geraden Abschnitte einen zweiten Winkel von 0° bis etwa 90° bildet, und/oder
wobei vorzugsweise bei Einführung der medizinischen Vorrichtung (10, 12) der Rahmen (806; 906; 1006; 1106) in Umfangsrichtung in einer Richtung expandiert, die dem einzelnen vertikalen Strebenelement (806; 906; 1006; 1106) entgegengesetzt ist, und wobei der Rahmen (806; 906; 1006; 1106) nicht entlang des einzelnen vertikalen Strebenelements (806; 906; 1006; 1106) expandiert.

9. Expandierbare Hülle (100, 300) zum Ausbringen einer medizinischen Vorrichtung (10, 12), umfassend:
wenigstens eine Vielzahl von Streben, die so angeordnet sind, dass sie einen expandierbaren zylindrischen Rahmen (1200; 1300; 1400; 1500) bilden, der ein proximales und ein distales Ende, eine Innenfläche und eine Außenfläche und eine Mittelachse (302), die sich entlang einer Länge des Rahmens (1200; 1300; 1400; 1500) erstreckt, aufweist;
wobei der expandierbare zylindrische Rahmen (1200; 1300; 1400; 1500) eine erste Länge (304) und eine gegenüberliegende zweite Länge (306) aufweist, und wobei in einer ersten Stellung wenigstens ein Abschnitt der ersten Länge (304) und wenigstens ein Abschnitt der zweiten Länge (304) im Wesentlichen gleich sind,
wobei der expandierbare zylindrische Rahmen (1200; 1300; 1400; 1500) von der ersten Stellung in eine zweite Stellung bewegbar ist, indem er sich von der Mittelachse (302) weg krümmt, wobei sich in der zweiten Stellung wenigstens ein Abschnitt der ersten Länge (304') verkürzt und wenigstens ein Abschnitt der zweiten Länge (306') verlängert;
wobei die Innenfläche des expandierbaren zylindrischen Rahmens (1200; 1300; 1400; 1500) ein Lumen definiert, das zur Aufnahme einer medizinischen Vorrichtung (10, 12) konfiguriert ist; und
wobei der expandierbare zylindrische Rahmen (1200; 1300; 1400; 1500) dazu konfiguriert ist, beim Passieren einer medizinischen Vorrichtung (10, 12) von einem ersten Durchmesser (d₁) in einer nicht expandierten Stellung auf einen zweiten Durchmesser (d₂) in einer expandierten Stellung zu expandieren; und wobei der expandierbare zylindrische Rahmen (1200; 1300; 1400; 1500) knickfest ist,
wobei die Vielzahl von Streben in einer Konfiguration angeordnet sind, in der jede Strebe der Vielzahl von Streben in der Lage ist, sich beim Einführen der medizinischen Vorrichtung (10, 12) individuell in einer Umfangsrichtung entlang der Länge des Rahmens (1200; 1300; 1400; 1500) zu verlängern, und
wobei die Vielzahl von Streben in einer Vielzahl von parallelen axialen Reihen (1220) angeordnet sind, und wobei jede Reihe (1220) der Vielzahl von parallelen axialen Reihen (1220) eine Wellenmustereinheit (1503) umfasst, wobei jede Einheit (1503) umfasst:
ein erstes Tal (1502), das einen im Wesentlichen geraden Bodenabschnitt aufweist, wobei der im Wesentlichen gerade Bodenabschnitt des ersten Tals (1502) eine erste Breite (w) aufweist,
wobei auf das erste Tal (1502) ein erster Scheitelpunkt (1508), ein zweites Tal (1504), ein zweiter Scheitelpunkt (1510) und ein drittes Tal (1506) folgen, und
wobei die Gesamtbreite des zweiten und des dritten Tals (1504, 1506) und des zweiten Scheitelpunkts (1510) im Wesentlichen gleich der ersten Breite (w) des im Wesentlichen geraden Bodenabschnitts des ersten Tals (1502) ist.

10. Expandierbare Hülle (100; 300) nach Anspruch 9,
wobei auf das dritte Tal (1506) ein dritter Scheitelpunkt (1512) und ein erstes Tal (1502) einer nächsten wellenförmigen Mustereinheit (1503) in derselben Reihe (1220) folgt, und/oder
wobei vorzugsweise die mehreren parallelen axialen Reihen so angeordnet sind, dass ein zweiter Scheitelpunkt in jeder sich wiederholenden Mustereinheit jeder Reihe über wenigstens einem Abschnitt eines im Wesentlichen geraden Bodenabschnitts eines ersten Tals einer benachbarten Reihe angeordnet und mit diesem durch ein Brückenelement gekoppelt ist.

11. Expandierbare Hülle (100; 300) nach einem der Ansprüche 1 bis 10, wobei das distale Ende des Rahmens (2800; 2900) eine vorgeformte Aufweitung umfasst, die dazu konfiguriert ist, in der nicht expandierten Stellung in einem komprimierten Zustand zu bleiben und beim Passieren der medizinischen Vorrichtung (10, 12) eine aufgeweitete Konfiguration anzunehmen,
wobei vorzugsweise die vorgeformte Aufweitung durch einen Faden (2802) in dem komprimierten Zustand gehalten wird, der an einer oder mehreren der mehreren Streben (2804) am distalen Ende des Rahmens angebracht ist und sich in Umfangsrichtung um die mehreren Streben (2804) am distalen Ende erstreckt, und
wobei, noch mehr bevorzugt, das distale Ende des Rahmens (2800; 2900) in der expandierten Stellung ist, der Faden (2802) an einer oder mehreren der mehreren Streben (2804) am distalen Ende des Rahmens angebracht bleibt, sich aber nicht in Umfangsrichtung um die mehreren Streben (2804) am distalen Ende erstreckt, so dass sich das distale Ende des Rahmens (2800; 2900) radial nach außen aufweiten kann.

12. Expandierbare Hülle (100; 300) nach einem der Ansprüche 1 bis 11, wobei der Rahmen dazu konfiguriert ist, sich beim Entfernen der medizinischen Vorrichtung (10, 12) auf einen Durchmesser zusammenzuziehen, der etwa 10% bis etwa 90% größer ist als der erste Durchmesser (d₁).

13. Expandierbare Hülle (100; 300) nach einem der Ansprüche 1 bis 12, wobei die mehreren Streben Nitinol, Edelstahl, Kobalt-Chrom-Legierungen, Polymer oder eine Kombination davon umfassen.

14. Expandierbare Hülle (100; 300) nach einem der Ansprüche 1 bis 13, wobei die Hülle ferner eine oder mehrere Polymerlagen (3004; 3004a) umfasst, wobei die eine oder die mehreren Polymerlagen (3004; 3004a) Polyethylen, Polypropylen, Polyetherblockamid (PEBAX^{®}), Silikon oder eine beliebige Kombination davon umfassen,
wobei vorzugsweise wenigstens ein Abschnitt des Rahmens in die eine oder die mehreren Polymerlagen (3004; 3004a) eingebettet ist, und
wobei, noch mehr bevorzugt, die eine oder die mehreren Polymerlagen (3004; 3004a) mehrere sich in Längsrichtung erstreckende Falten umfassen, wobei die Hülle den ersten Durchmesser (d₁) aufweist, und wobei die sich in Längsrichtung erstreckenden Falten mehrere in Umfangsrichtung beabstandete Rippen (3004c) und mehrere in Umfangsrichtung beabstandete Täler (3004b) erzeugen, und
wobei vorzugsweise dann, wenn die medizinische Vorrichtung (10, 12) durch die Hülle hindurchgeht, die Rippen (3004c) und Täler (3004b) sich ausgleichen, damit die Hülle radial expandieren kann.

15. Expandierbare Hülle (100; 300) nach Anspruch 14, wobei die eine oder die mehreren Polymerlagen (3004; 3004a) eine erste Dicke aufweisen, bei der die Hülle den ersten Durchmesser (d₁) hat, und wobei die eine oder die mehreren Polymerlagen (3004; 3004a) beim Passieren der medizinischen Vorrichtung (10, 12) durch die Hülle auf eine zweite Dicke verdünnt werden, damit die Hülle radial expandieren kann.

## Revendications

1. Gaine extensible (100 ; 300) pour le déploiement d'un dispositif médical (10, 12) comprenant :
au moins une pluralité d'entretoises agencées pour former un cadre (500 ; 600 ; 800 ; 900 ; 1000 ; 1100 ; 1800 ; 1900) cylindrique extensible présentant une extrémité proximale et une extrémité distale, une surface interne et une surface externe et un axe central (302) s'étendant le long d'une longueur du cadre (500 ; 600 ; 800 ; 900 ; 1000 ; 1100 ; 1800 ; 1900) ;
la pluralité d'entretoises étant agencées pour former une configuration en zigzag comprenant une pluralité de parties sensiblement droites (502, 502a ; 602, 602a ; 802, 802a) suivies circonférentiellement par une pluralité de parties sensiblement courbées, chaque partie droite (502, 502a ; 602, 602a ; 802, 802a) étant suivie d'une partie courbée (504 ; 604 ; 818) ;
le cadre (500 ; 600 ; 800 ; 900 ; 1000 ; 1100 ; 1800 ; 1900) cylindrique extensible présentant une première longueur (304) et une seconde longueur (306) opposée et, dans une première position, au moins une partie de la première longueur (304) et au moins une partie de la seconde longueur (306) étant sensiblement les mêmes,
le cadre (500 ; 600 ; 800 ; 900 ; 1000 ; 1100 ; 1800 ; 1900) cylindrique extensible étant mobile à partir de la première position vers une seconde position en s'incurvant à l'opposé de l'axe central (302), au moins une partie de la première longueur (304') se raccourcissant et au moins une partie de la seconde longueur (306') s'allongeant dans la seconde position ;
la surface interne du cadre (500 ; 600 ; 800 ; 900 ; 1000 ; 1100 ; 1800 ; 1900) cylindrique extensible définissant une lumière conçue pour recevoir un dispositif médical (10, 12) ; et
le cadre (500 ; 600 ; 800 ; 900 ; 1000 ; 1100 ; 1800 ; 1900) cylindrique extensible étant conçu pour s'étendre à partir d'un premier diamètre (d₁) dans une position non étendue vers un second diamètre (d₂) dans une position étendue lors du passage d'un dispositif médical (10, 12) ; et le cadre (500 ; 600 ; 800 ; 900 ; 1000 ; 1100 ; 1800 ; 1900) cylindrique extensible étant résistant à la formation de coudes.

2. Gaine extensible (100 ; 300) selon la revendication 1, la partie de la première longueur et la partie de la seconde longueur (306') dans la seconde position étant situées le long d'une partie incurvée du cadre (500 ; 600 ; 800 ; 900 ; 1000 ; 1100 ; 1800 ; 1900) et/ou
de préférence, la première longueur (304, 304') étant prévue sur une première partie d'un diamètre externe du cadre (500 ; 600 ; 800 ; 900 ; 1000 ; 1100 ; 1800 ; 1900) cylindrique extensible et la seconde longueur (306, 306') étant prévue sur une seconde partie du diamètre externe du cadre (500 ; 600 ; 800 ; 900 ; 1000 ; 1100 ; 1800 ; 1900) cylindrique extensible, la première partie étant circonférentiellement opposée à la seconde partie du diamètre externe et/ou
en outre de préférence, le premier diamètre (d₁) étant d'environ 3 mm à environ 5 mm et le second diamètre (d₂) étant d'environ 5 mm à environ 10 mm.

3. Gaine extensible (100 ; 300) selon l'une quelconque des revendications 1 ou 2, la configuration en zigzag étant enroulée le long d'un axe central (302) afin de former le cadre (500 ; 600) ou
la configuration en zigzag formant une pluralité de rangées circonférentielles parallèles (816) ; et la pluralité de rangées circonférentielles parallèles (816) étant accouplées axialement les unes aux autres par un élément d'entretoise vertical unique (806 ; 906 ; 1006 ; 1106) disposé le long d'une longueur du cadre (800 ; 900 ; 1000 ; 1100) ou
la configuration en zigzag formant une pluralité de rangées circonférentielles parallèles (1920) ; des rangées adjacentes de la pluralité de rangées circonférentielles parallèles (1920) étant accouplées ensemble par un élément de pontage (1806 ; 1906) unique s'étendant à partir d'une partie sensiblement courbée dans une première rangée vers une partie sensiblement courbée correspondante dans une seconde rangée (1920) adjacente et l'élément de pontage (1806 ; 1906) unique accouplant deux rangées adjacentes (1920) quelconques étant décalé circonférentiellement par rapport à un élément de pontage (1806 ; 1906) accouplant l'une quelconque des deux rangées adjacentes (1920) suivantes.

4. Gaine extensible (100 ; 300) selon la revendication 3, chaque partie sensiblement droite (502, 502a ; 602, 602a ; 802, 802a) de la pluralité de parties sensiblement droites (502, 502a ; 602, 602a ; 802, 802a) étant de longueur égale.

5. Gaine extensible selon la revendication 3, la pluralité de parties sensiblement droites (502, 502a ; 602, 602a ; 802, 802a) comprenant une ou plusieurs parties présentant des longueurs inégales au niveau des extrémités distale et/ou proximale du cadre.

6. Gaine extensible (100 ; 300) selon l'une quelconque des revendications 3 à 5, la pluralité de parties sensiblement courbées (504 ; 604 ; 818) définissant une ligne bissectrice s'étendant à travers un sommet de la pluralité de parties sensiblement courbées (504 ; 604 ; 818) et s'étendant longitudinalement le long d'une longueur de la gaine et chaque partie sensiblement droite (502, 502a ; 602, 602a ; 802, 802a) de la pluralité de parties sensiblement droites (502, 502a ; 602, 602a ; 802, 802a) présentant un premier angle par rapport à la ligne bissectrice de 0° à environ 90°.

7. Gaine extensible (100 ; 300) selon la revendication 6, le cadre étant dans la position étendue, le premier angle augmentant de plus de 0 % à environ 600 % de sa valeur d'origine.

8. Gaine extensible (100 ; 300) selon l'une quelconque des revendications 3 à 7, l'élément d'entretoise vertical (806 ; 906 ; 1006 ; 1106) unique s'étendant longitudinalement le long d'une longueur de la gaine et reliant les sommets des parties sensiblement courbées de chaque rangée de la pluralité de rangées circonférentielles parallèles et l'élément d'entretoise vertical formant un second angle avec chaque partie sensiblement droite des parties sensiblement droites adjacentes correspondantes de 0° à environ 90° et/ou
de préférence, lors de l'introduction du dispositif médical (10, 12), le cadre (806 ; 906 ; 1006 ; 1106) s'étendant circonférentiellement dans une direction opposée à l'élément d'entretoise vertical (806 ; 906 ; 1006 ; 1106) unique et le cadre (806 ; 906 ; 1006 ; 1106) ne s'étendant pas le long de l'élément d'entretoise vertical (806 ; 906 ; 1006 ; 1106) unique.

9. Gaine extensible (100 ; 300) pour le déploiement d'un dispositif médical (10, 12) comprenant :
au moins une pluralité d'entretoises agencées pour former un cadre (1200 ; 1300 ; 1400 ; 1500) cylindrique extensible présentant une extrémité proximale et une extrémité distale, une surface interne et une surface externe et un axe central (302) s'étendant le long d'une longueur du cadre (1200 ; 1300 ; 1400 ; 1500) ;
le cadre (1200 ; 1300 ; 1400 ; 1500) cylindrique extensible présentant une première longueur (304) et une seconde longueur (306) opposée et, dans une première position, au moins une partie de la première longueur (304) et au moins une partie de la seconde longueur (304) étant sensiblement les mêmes,
le cadre (1200 ; 1300 ; 1400 ; 1500) cylindrique extensible étant mobile à partir de la première position vers une seconde position en s'incurvant à l'opposé de l'axe central (302), au moins une partie de la première longueur (304') se raccourcissant et au moins une partie de la seconde longueur (306') s'allongeant dans la seconde position ;
la surface interne du cadre (1200 ; 1300 ; 1400 ; 1500) cylindrique extensible définissant une lumière conçue pour recevoir un dispositif médical (10, 12) ; et
le cadre (1200 ; 1300 ; 1400 ; 1500) cylindrique extensible étant conçu pour s'étendre à partir d'un premier diamètre (d₁) dans une position non étendue vers un second diamètre (d₂) dans une position étendue lors du passage d'un dispositif médical (10, 12) ; et le cadre (1200 ; 1300 ; 1400 ; 1500) cylindrique extensible étant résistant à la formation de coudes,
la pluralité d'entretoises étant agencées dans une configuration dans laquelle chaque entretoise de la pluralité d'entretoises peut s'allonger individuellement dans une direction circonférentielle le long de la longueur du cadre (1200 ; 1300 ; 1400 ; 1500) lors de l'introduction du dispositif médical (10, 12) et
la pluralité d'entretoises étant agencées en une pluralité de rangées axiales parallèles (1220) et chaque rangée (1220) de la pluralité de rangées axiales parallèles (1220) comprenant une unité de motif ondulé (1503), chaque unité (1503) comprenant :
un premier creux (1502) présentant une partie inférieure sensiblement droite, la partie inférieure sensiblement droite du premier creux (1502) présentant une première largeur (w),
le premier creux (1502) étant suivi d'un premier sommet (1508), d'un deuxième creux (1504), d'un deuxième sommet (1510) et d'un troisième creux (1506) et
une largeur totale des deuxième et troisième creux (1504, 1506) et du deuxième sommet (1510) étant sensiblement la même que la première largeur (w) de la partie inférieure sensiblement droite du premier creux (1502).

10. Gaine extensible (100 ; 300) selon la revendication 9, le troisième creux (1506) étant suivi d'un troisième sommet (1512) et d'un premier creux (1502) d'une unité de motif ondulé suivante (1503) dans la même rangée (1220) et/ou
de préférence, la pluralité de rangées axiales parallèles étant agencées de telle sorte qu'un deuxième sommet dans chaque unité de motif répétitif de chaque rangée est positionné au-dessus d'au moins une partie d'une partie inférieure sensiblement droite d'un premier creux d'une rangée adjacente et accouplé à celle-ci par un élément de pontage.

11. Gaine extensible (100 ; 300) selon l'une quelconque des revendications 1 à 10, l'extrémité distale du cadre (2800 ; 2900) comprenant un évasement préformé qui est conçu pour rester dans un état comprimé dans la position non étendue et pour adopter une configuration évasée lors du passage du dispositif médical (10, 12),
de préférence, l'évasement préformé étant maintenu dans l'état comprimé par un filament (2802) attaché à une ou plusieurs entretoises parmi la pluralité d'entretoises (2804) au niveau de l'extrémité distale du cadre et s'étendant circonférentiellement autour de la pluralité d'entretoises (2804) au niveau de l'extrémité distale et
en outre de préférence, l'extrémité distale du cadre (2800 ; 2900) étant dans la position étendue, le filament (2802) restant attaché à une ou plusieurs entretoises parmi la pluralité d'entretoises (2804) au niveau de l'extrémité distale du cadre mais ne s'étendant pas circonférentiellement autour de la pluralité d'entretoises (2804) au niveau de l'extrémité distale, permettant ainsi à l'extrémité distale du cadre (2800 ; 2900) de s'évaser radialement vers l'extérieur.

12. Gaine extensible (100 ; 300) selon l'une quelconque des revendications 1 à 11, le cadre étant conçu pour se contracter à un diamètre qui est d'environ 10% à environ 90% plus grand que le premier diamètre (d₁) lors du retrait du dispositif médical (10, 12).

13. Gaine extensible (100 ; 300) selon l'une quelconque des revendications 1 à 12, la pluralité d'entretoises comprenant du nitinol, de l'acier inoxydable, des alliages cobalt-chrome, un polymère ou une quelconque combinaison de ceux-ci.

14. Gaine extensible (100 ; 300) selon l'une quelconque des revendications 1 à 13, la gaine comprenant en outre une ou plusieurs couches polymères (3004 ; 3004a), ladite une ou lesdites plusieurs couches polymères (3004 ; 3004a) comprenant du polyéthylène, du polypropylène, du polyéther-bloc-amide (PEBAX^{®}), de la silicone ou une quelconque combinaison de ceux-ci,
de préférence, au moins une partie du cadre étant incorporée à l'intérieur de ladite une ou desdites plusieurs couches polymères (3004 ; 3004a) et
en outre de préférence, ladite une ou lesdites plusieurs couches polymères (3004 ; 3004a) comprenant une pluralité de plis s'étendant longitudinalement, la gaine étant au premier diamètre (d₁) et les plis s'étendant longitudinalement créant une pluralité de crêtes (3004c) espacées circonférentiellement et une pluralité de creux (3004b) espacés circonférentiellement et
de préférence, lorsque le dispositif médical (10, 12) passe à travers la gaine, les crêtes (3004c) et les creux (3004b) s'aplanissent afin de permettre à la gaine de s'étendre radialement.

15. Gaine extensible (100 ; 300) selon la revendication 14, ladite une ou lesdites plusieurs couches polymères (3004 ; 3004a) présentant une première épaisseur, la gaine étant au premier diamètre (d₁) et, lorsque le dispositif médical (10, 12) passe à travers la gaine, ladite une ou lesdites plusieurs couches polymères (3004 ; 3004a) s'amincissent à une seconde épaisseur afin de permettre à la gaine de s'étendre radialement.
